# EUROPEAN PATENT APPLICATION

(11) **EP 4 733 294 A1**
(43) Date of publication of application: **29.04.2026**
(21) Application number: 24306802.0
(22) Date of filing: 25.10.2024
(51) Int. Cl.: C07C 209/34, C07C 209/36, C07C 213/02, C07C 221/00, B01J 27/14, B01J 23/755, C07C 239/10, C07C 239/12, C07C 239/14, C07C 253/30, C07F 15/04

(54) **NOVEL PROCESS**

(71) Applicant: Oxford University Innovation Limited, Oxford, OX2 0JB (GB)
(72) Inventor: The designation of the inventor has not yet been filed
(74) Representative: J A Kemp LLP

(57) **Abstract**

Provided herein is a method of reducing a nitrogen-containing functional group in a target compound using a Ni catalyst. Also provided are related systems.

## Description

### FIELD

Provided herein is a method of reducing a nitrogen-containing functional group in a target compound using a Ni catalyst as described in more detail herein. Also provided are related systems.

### BACKGROUND

One key class of chemical reaction which is particularly important in synthesis is the reduction of nitrogen-containing functional groups such as nitro groups, hydroxylamine groups and the like.

The reduction of nitro-compounds towards the corresponding amine is a reaction of immense industrial importance as a key method for amine synthesis in the production of pharmaceuticals, polyurethanes, and pesticides. A range of processes employing stoichiometric reagents are known, including notably the Bechamp reduction (using Fe and hydrochloric acid) and treatment with tin(II) chloride.

In addition, hydrogenation of nitro-compounds to hydroxylamine compounds is also important as such compounds find uses including for example as polymerisation inhibitors and as precursors in fine chemical synthesis.

Chemical manufacturing processes are typically associated with many environmental concerns. The reaction conditions necessary are typically harsh, employing elevated temperatures and pressure and/or hazardous organic solvents. Furthermore, such reactions often generate significant side-products e.g. arising from non-specific reduction, incomplete reduction, and catalysis of unwanted side-reactions such as bond rearrangements.

The non-selectivity of conventional processes is particularly problematic in the field of fine chemical manufacture, including in the synthesis of pharmaceuticals, where it is particularly important to minimise production of impurities. Such impurities typically need to be removed through purification strategies, but such purification methods are

costly and typically inefficient. Accordingly, the generation of unwanted by-products in chemical reactions increases financial costs and leads to environmental damage as the chemicals used in purification processes are typically also damaging.

Hence, there is a need for alternative or improved processes of catalysing the reduction of nitrogen-containing functional groups (such as nitro functional groups and/or hydroxylamine functional groups). In particular, there is a need for methods that avoid the requirement for expensive or dangerous chemicals; that are scalable; that are selective; and/or that avoid the generation of by-products. The present invention aims to address some or all of these problems.

In seeking to address the above problems, the present inventors have surprisingly found that target compounds containing nitrogen-containing functional groups (such as nitro functional groups and hydroxylamine functional groups) can be cleanly and efficiently reduced by the use of an Ni catalyst in the presence of a molecular reductant such as hydrogen. Typically, the processes are selective such that nitrogen-containing functional groups (such as nitro functional groups and hydroxylamine functional groups) can be reduced in the presence of other sensitive groups such as C=C double bonds.

### SUMMARY

A first aspect provides a process of reducing a nitrogen-containing functional group in a target compound, comprising contacting the target compound with a catalyst of Formula (I) in the presence of a molecular reductant: 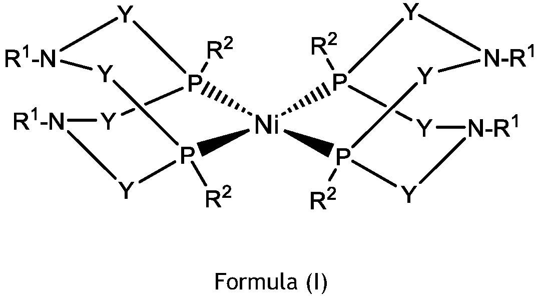 wherein:
Y, each independently, is -C(R³)₂-; and R³, each independently, is H or C₁₋₃ alkyl; R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
R², each independently, is a 5-6 membered cyclic group, wherein the cyclic group may be optionally substituted, and wherein the cyclic group may optionally include one or more heteroatoms N, O or S in its carbon skeleton.

A second aspect provides a system comprising:
(i) a catalyst of Formula (I) 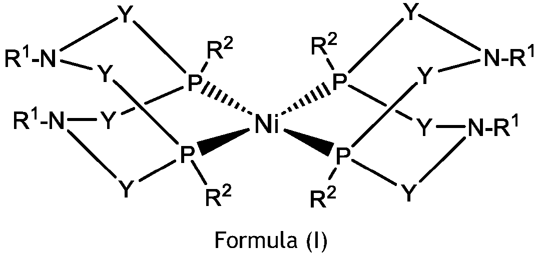 wherein:
   Y, each independently, is C(R³)_{2;} and R³, each independently, is H or C₁₋₃ alkyl;
   R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
   R², each independently, is a 5-6 membered cyclic group, wherein the cyclic group may be optionally substituted, and wherein the cyclic group may optionally include one or more heteroatoms N, O or S in its carbon skeleton;
(ii) a molecular reductant; and
(iii) a target compound comprising a nitrogen-containing functional group;
wherein the system is configured such that, in use, the nitrogen-containing functional group is reduced.

### BRIEF DESCRIPTION OF THE FIGURES

Figure 1 shows nuclear magnetic resonance spectroscopy (NMR) spectra comparing (from top to bottom): (i) aniline; (ii) N-phenylhydroxylamine (PHA); (iii) the reaction product after hydrogenation treatment of nitrobenzene in the presence of NiArg catalyst for 24 hours; (iv) the reaction product after hydrogenation treatment of nitrobenzene in the presence of NiArg catalyst for 48 hours; and (v) the reaction product after hydrogenation treatment of nitrobenzene in the presence of NiArg/C catalyst for 24 hours.
Figure 2 is a graph showing the relative integral (as measured by 1H NMR) of nitrobenzene (hollow dots), N-phenylhydroxylamine (PHA) (filled triangles) and aniline (filled hexagon) as measured over 24 hours for a hydrogenation of nitrobenzene in the presence of NiArg/C.
Figure 3 is a graph showing the relative integral (as measured by 1H NMR) of nitrobenzene (hollow dots), N-phenylhydroxylamine (PHA)(filled triangles) and aniline (filled hexagon) as measured over 24 hours for a hydrogenation of nitrobenzene in the presence of NiArg.
Figure 4 shows nuclear magnetic resonance spectroscopy (NMR) spectra comparing (from top to bottom): (i) nitrohexane; (ii) hexylamine; and (iii) the reaction product after hydrogenation treatment of nitrohexane in the presence of NiArg/C catalyst for 24 hours.
Figure 5 shows nuclear magnetic resonance spectroscopy (NMR) spectra comparing (from top to bottom): (i) cyclo-nitrohexane; (ii) cyclo-hexylamine; and (iii) the reaction product after hydrogenation treatment of cyclo-nitrohexane in the presence of NiArg/NCNT catalyst for 24 hours.
Figure 6 shows nuclear magnetic resonance spectroscopy (NMR) spectra comparing (from top to bottom): (i) nitrobenzene; (ii) aniline; and (iii) the reaction product after hydrogenation treatment of nitrobenzene in the presence of NiArg/C catalyst (from a one-pot preparation) for 24 hours.
Figure 7 is a cyclic voltammogram for NiArg complex adsorbed onto a pyrolytic graphite edge-plane electrode under H₂ and electrode rotation at 3000 RPM, scan rate 1 mV/s. Dashed vertical line represents E'(2H⁺/H₂) at pH 6 and 1 bar H₂. Hydrogen oxidation catalysed by NiArg complex commences with very low overpotential.

### DETAILED DESCRIPTION

### Process

A first aspect provides a process of reducing a nitrogen-containing functional group in a target compound, comprising contacting the target compound with a catalyst of Formula (I) in the presence of a molecular reductant: 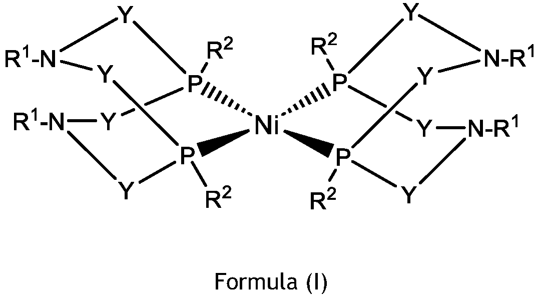 wherein:
Y, each independently, is -C(R³)₂-; and R³, each independently, is H or C₁₋₃ alkyl;
R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
R², each independently, is a 5-6 membered cyclic group, wherein the cyclic group may be optionally substituted, and wherein the cyclic group may optionally include one or more heteroatoms N, O or S in its carbon skeleton.

In one embodiment, the process is a process of reducing a nitrogen-containing functional group selected from the group consisting of nitro, hydroxylamine, azido, azo, azoxy and nitroso.

In one embodiment, the process is a process of reducing a nitro functional group and/or a hydroxylamine functional group.

In one embodiment, the process is a process of reducing a nitro functional group.

In one embodiment, the process is a process of reducing a hydroxylamine functional group.

In one embodiment, the catalyst is used in heterogeneous form. Performing the process with the catalyst in heterogeneous form may lead to easier removal of the catalyst from the reaction mixture and hence easier purification of the product from the reaction mixture. Typically, the catalyst may be used in heterogeneous form supported on a support material.

In one embodiment, the catalyst is used in homogeneous form.

When the process is a process of reducing a nitro functional group in a target compound, the reduced product prepared may be an amine, a hydroxylamine or a mixture thereof. For example, there may be conversion to the amine. Or for example, there may be conversion to the hydroxylamine. Alternatively, the product may be obtained with a proportion of the molecules converted to the amine and a proportion of molecules converted to the hydroxylamine.

The process conditions may be modified to adjust the proportion of amine and/or hydroxylamine obtained as desired.

Typically, when the process is a process of reducing a nitro functional group in a target compound, and the product is an amine, the catalyst may be used in heterogeneous form supported on a support material, with weight ratio of the support material to the catalyst of 40 or more:1. Typically, at least 50 mol%, e.g. at least 60 mol%, at least 70 mol%, at least 80 mol%, at least 90 mol%, at least 95 mol%, at least 97 mol%, at least 98 mol%, at least 99 mol %, at least 99.9 mol % or at least 99.99 mol % of the nitro functional groups in the target compounds in the sample may be completely reduced to an amine.

Typically, when the process is a process of reducing a nitro functional group in a target compound, and the product is a hydroxylamine, the catalyst may be used in homogeneous form or in heterogeneous form on a support material with a weight ratio of the support material to the catalyst of 39 or less:1. Typically, with a weight ratio of the support material to the catalyst of 30 or less: 1. More typically, with a weight ratio of the support material to the catalyst of 10 or less: 1. Typically, at least 50 mol%, e.g. at least 60 mol%, at least 70 mol%, at least 80 mol%, at least 90 mol%, at least 95 mol%, at least 97 mol%, at least 98 mol%, at least 99 mol %, at least 99.9 mol % or at least 99.99 mol % of the nitro functional groups in the target compounds in the sample may be completely reduced to a hydroxylamine.

The processes disclosed herein are typically highly selective for the functional group to be reduced. Typically, the processes disclosed herein are selective for reduction of the nitrogen-containing functional group (e.g. the nitro or hydroxylamine functional group) even in the presence of a C=C double bond. For example, the processes may comprise contacting a target compound comprising a nitrogen-containing functional group (such as a nitro or hydroxylamine functional group) and a C=C double bond with a catalyst as described herein. In such embodiments, typically the nitrogen-containing functional group (e.g. the nitro or hydroxylamine functional group) is reduced and the C=C double bond is not reduced. This is described in more detail in the examples. This may represent a particular advantage of the disclosed processes, as such selective reduction is typically not possible with palladium catalysts.

### Catalyst

Described herein is a catalyst of Formula (I): 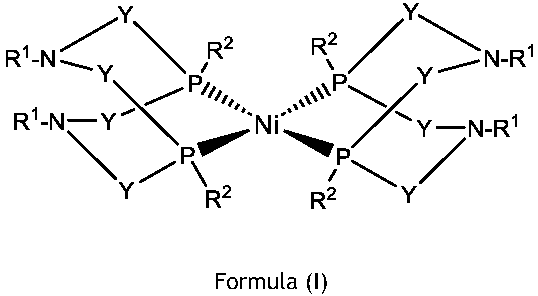 wherein:
Y, each independently, is -C(R³)₂-; and R³, each independently, is H or C₁₋₃ alkyl;
R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
R², each independently, is a 5-6 membered cyclic group, wherein the cyclic group may be optionally substituted, and wherein the cyclic group may optionally include one or more heteroatoms N, O or S in its carbon skeleton.

In one embodiment, at least 1 Y is-CH₂-. Typically, 2, 3, 4, 5, 6 or 7 Y is -CH₂. Most typically, each Y is -CH₂-.

In one embodiment, 1, 2 or 3 R¹ are the same. Typically, each R¹ is the same.

In one embodiment R¹, is a saturated or unsaturated C₁-C₂₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton. Typically, the hydrocarbyl includes up to one heteroatom in its carbon skeleton. More typically, the hydrocarbyl includes no heteroatoms in its carbon skeleton. More typically, the hydrocarbyl includes 0, 1 or 2 substituents.

In one embodiment, R¹ is selected from the group consisting of a group of Formula (II), aryl and alkylaryl, wherein any group may be substituted with 0, 1 or 2 substituents. Typically, R¹ is selected from the group consisting of a group of Formula (II), aryl and alkylaryl, wherein any group may be substituted with 0, 1 or 2 substituents; wherein aryl is selected from the group consisting of phenyl, biphenyl, anthracenyl, phenanthrenyl, pyrenyl and naphthalenyl; and alkyl is C₁-C₄alkyl. More typically, R¹ is selected from the group consisting of argininyl, -CH₂-pyrenyl and pyrenyl. More typically, R¹ is argininyl or -CH₂-pyrenyl. More typically, R¹ is argininyl.

As used herein argininyl refers to:

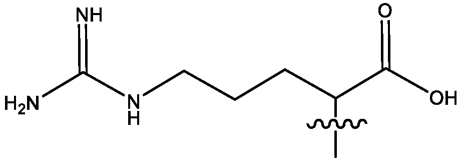

In one embodiment, at least one R¹ is a group of Formula (II): 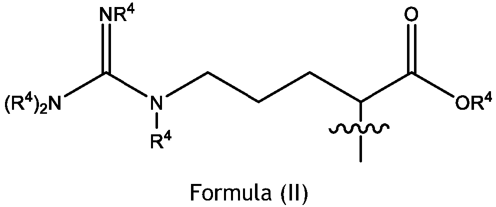 wherein R⁴, each independently, is H or C₁₋₃ alkyl; optionally wherein each R⁴ is H.

In one embodiment, at least one R¹ comprises a guanidino group; optionally wherein at least one R¹ is argininyl.

In one embodiment, R¹ comprises an aryl or heteroaryl group. Typically, R¹ is alkylaryl or aryl. Typically, R¹ is -CH₂-aryl or aryl. Typically, aryl is selected from the group consisting of phenyl, biphenyl, anthracenyl, phenanthrenyl, pyrenyl and naphthalenyl. Typically, at least one R¹ is -CH₂-pyrenyl or pyrenyl. More typically, R¹ is -CH₂-pyrenyl.

As used herein -CH₂-pyrenyl refers to:

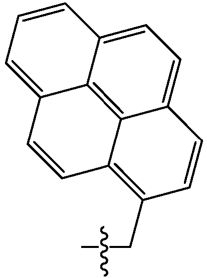

In one embodiment R², is a 5-6 membered cyclic group and may optionally include up to one heteroatom N, O or S in its carbon skeleton. Typically, R₂ has no heteroatoms in its carbon skeleton.

In one embodiment, R₂ is cyclohexyl or phenyl; optionally substituted with up to one substituent. Typically, R₂ is cyclohexyl or phenyl. More typically, R₂ is cyclohexyl.

In one embodiment, at least 1 R₂ is cyclohexyl. Typically, 2 or 3 R₂ is cyclohexyl. Most typically, each R₂ is cyclohexyl.

In one embodiment, 1, 2 or 3 R₂ are the same. Typically, each R₂ is the same.

Substituents are typically selected from the group consisting of F, Br, Cl, I, CN, OR⁵, N(R⁵)₂, -N(R⁵)-C(=NR⁵)-NR⁵₂, SR⁵, SOR⁵, SO₂R⁵, C(O)R⁵, C(O)OR⁵, and C(O)NR⁵₂, C₁ to C₆ (e.g. C₁ to C₃) alkyl groups, C₁ to C₆ (e.g. C₁ to C₃) alkyl groups substituted with 1, 2 or 3 halogens (e.g. F, Br, Cl, I), -CH=CH₂ and -C≡CH; wherein R⁵ is H or C₁ to C₃ alkyl. More typically, substituents are selected from the group consisting of F, Br, Cl, I, -N(R⁵)-C(=NR⁵)-NR⁵₂, C₁ to C₆ (e.g. C₁ to C₃) alkyl groups, -CH=CH₂ and -C≡CH; wherein R⁵ is H or C₁ to C₃ alkyl. More typically, substituents are selected from the group consisting of F, Br, Cl, I, C₁ to C₆ (e.g. C₁ to C₃) alkyl groups, wherein R⁵ is H or C₁ to C₃ alkyl. More typically, the substituent is -N(R⁵)-C(=NR⁵)-NR⁵₂, wherein R⁵ is H or C₁ to C₃ alkyl. Typically, a substituted group comprises 1, 2, 3 or 4 substituents, more typically 1, 2 or 3 substituents, more typically 1 or 2 substituents, and more typically 1 substituent.

In one embodiment, the catalyst of Formula (I) is:

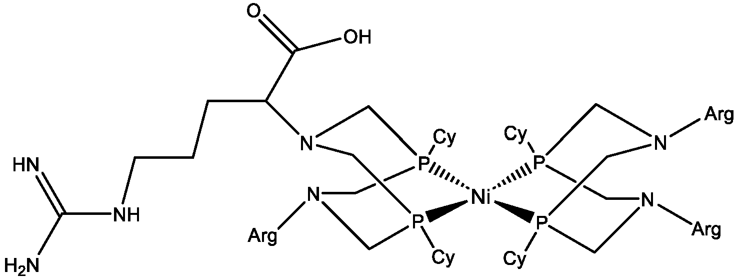

The catalyst directly above is referred to herein in as NiArg.

Dutta, A.; Roberts, J. A. S.; Shaw, W. J. Arginine-Containing Ligands Enhance H2 Oxidation Catalyst Performance. Angewandte Chemie International Edition 2014, 53 (25), 6487-6491. DOI: https://doi.org/10.1002/anie.201402304 describes NiArg.

For the avoidance of doubt, Arg refers to:

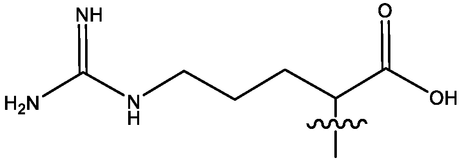

For the avoidance of doubt, Cy refers to cyclohexyl.

In one embodiment, the catalyst of Formula (I) is:

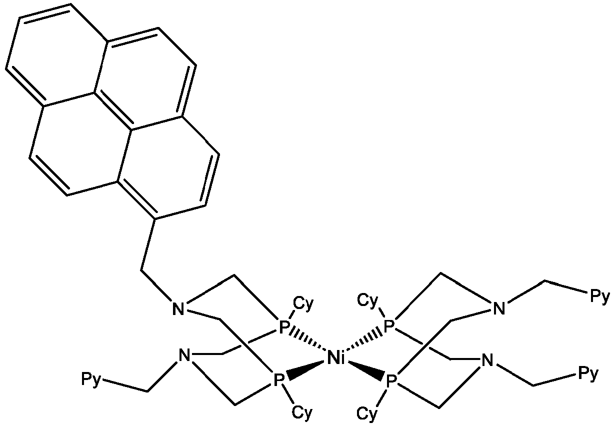

The catalyst directly above is referred to herein in as Ni(-CH₂Py).

The synthesis of the Ni(-CH₂Py) complex ([Ni(P₂^{Cy}N^{-CH2Pyrene})₂](BF₄)₂ and its immobilisation on carbon is known and is reported here: Tran, P. D.; Le Goff, A.; Heidkamp, J.; Jousselme, B.; Guillet, N.; Palacin, S.; Dau, H.; Fontecave, M.; Artero, V. Noncovalent Modification of Carbon Nanotubes with Pyrene-Functionalized Nickel Complexes: Carbon Monoxide Tolerant Catalysts for Hydrogen Evolution and Uptake. Angewandte Chemie International Edition 2011, 50 (6), 1371-1374. DOI: https://doi.org/10.1002/anie.201005427

For the avoidance of doubt, as used in the catalyst directly above, -CHz-Py refers to:

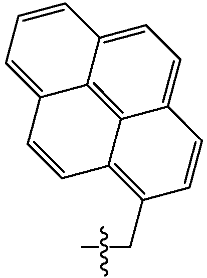

The Turnover Frequency (TOF) is a measure of the number of moles of product generated per second per mole of catalyst present. Preferably, in the processes of the invention, the catalyst has a TOF of 0.001 s⁻¹ to 0.1 s⁻¹.

In one embodiment, the catalyst may be prepared and then used in the process without being isolated between the preparation and use in the process.

### Support material

In one embodiment, the catalyst is supported on a support material. Typically, the support material is electronically conductive or semi-conductive. Typically, the support material comprises carbon, a metal or metal alloy, a metal oxide or mixed metal oxide, a metal hydroxide, a metal chalcogenide, a semi-conducting material, or an electronically-conductive polymer, or mixtures thereof.

When the catalyst is on a support the name of the support is included in the name. For example, NiArg/C refers to NiArg supported on carbon.

A benefit which arises from the support of the catalyst is that the supported catalyst can be easily removed from the reaction mixture. For example, the support(s) can be removed by sedimentation, filtration, centrifugation, or the like. Many such processes are known to those skilled in the art, e.g. filtration can be achieved using a simple filter paper to remove solid components from a liquid composition; or a mixed solid/liquid composition can be allowed to settle and the liquid then decanted from the settled solids.

In one embodiment, the catalyst is supported on a support material, wherein the support material comprises or consists of a carbon material. Typically, the support material comprises a carbon material. Typically, the carbon material comprises graphite, carbon nanotube(s), carbon black, activated carbon, carbon nanopowder, vitreous carbon, carbon fibre(s), carbon cloth, carbon felt, carbon paper, graphene, highly oriented pyrolytic graphite, pyrolytic graphite, carbon quantum dots, doped or surface-modified carbon or doped diamond. Typically, the carbon material comprises:
- doped graphene, wherein said graphene is doped with one or more dopants selected from nitrogen, boron, sulphur, oxygen, silicon, phosphorus, lanthanide elements and transition-metals;
- doped carbon nanotube(s), wherein said carbon nanotube(s) are doped with one or more dopants selected from nitrogen, boron, sulphur, oxygen, silicon, phosphorus, lanthanide elements and transition-metals;
- doped diamond, wherein said diamond is doped with one or more dopants selected from nitrogen, boron, sulphur, oxygen, silicon and phosphorus;
- doped carbon black, wherein said carbon black is doped with one or more dopants selected from nitrogen, boron, sulphur, oxygen, silicon, phosphorus, lanthanide elements and transition-metals;
- doped activated carbon, wherein said activated carbon is doped with one or more dopants selected from nitrogen, boron, sulphur, oxygen, silicon, phosphorus, lanthanide elements and transition-metals; and/or
- doped carbon quantum dots, wherein said carbon quantum dots are doped with one or more dopants selected from nitrogen, boron, sulphur, oxygen, silicon, phosphorus, lanthanide elements and transition-metals;
and/or wherein said carbon material comprises or is modified to comprise carboxylic acid surface groups. More typically, the carbon material comprises carbon black or nitrogen-doped carbon nanotubes. Most typically, the carbon material comprises carbon black.

In one embodiment, the catalyst is immobilized on a support material. As used herein, the term "immobilized" embraces adsorption, and/or cross-linkage between the support and the catalyst. Adsorption embraces non-covalent interactions including electrostatic interactions, hydrophobic interactions, and the like. Cross-linkage embraces covalent attachment, either directly between the catalyst and the support (e.g. via amide coupling, such as via EDC/NHS and/or other coupling agents routine to those skilled in the art) or using one or more covalent cross-linkers such as thiol-terminated linkers or crosslinking reagents. Typically, the catalyst is adsorbed or covalently attached to the support material. More typically, the catalyst is adsorbed to the support material. Combinations of some or all of the above mentioned immobilization means may be used.

Non-covalent immobilisation of catalysts onto carbon is described for example here: Ghedjatti, A.; Coutard, N.; Calvillo, L.; Granozzi, G.; Reuillard, B.; Artero, V.; Guetaz, L.; Lyonnard, S.; Okuno, H.; Chenevier, P. How do H2 oxidation molecular catalysts assemble onto carbon nanotube electrodes? A crosstalk between electrochemical and multi-physical characterization techniques. Chemical Science 2021, 12 (48), 15916-15927, 10.1039/D1SC05168G. DOI: 10.1039/D1SC05168G; and here:
Reuillard, B.; Blanco, M.; Calvillo, L.; Coutard, N.; Ghedjatti, A.; Chenevier, P.; Agnoli, S.; Otyepka, M.; Granozzi, G.; Artero, V. Noncovalent Integration of a Bioinspired Ni Catalyst to Graphene Acid for Reversible Electrocatalytic Hydrogen Oxidation. ACS Appl Mater Interfaces 2020, 12 (5), 5805-5811. DOI: 10.1021/acsami.9b18922.

Preferably, the or each support independently comprises a material comprising carbon (including doped carbon materials), a metal or metal alloy, a metal oxide (including mixed metal oxides), a metal hydroxide (including layered double hydroxides), a metal chalcogenide, a semi-conducting material (including carbon nitrides, silicates, germanium compounds and gallium compounds such as silicon carbide, doped silicon and/or doped germanium) or an electronically-conductive polymer; or mixtures thereof.

As those skilled in the art will appreciate, suitable support materials can include mixtures of materials described herein. As used herein, the term "mixture" embraces both atomic mixtures such as alloys, and heterogeneous mixtures such as mixtures of particles of one or more materials disclosed herein.

Examples of doped carbon materials include carbon doped with boron, nitrogen, oxygen, phosphorus, silicon, sulfur, first- or second-row transition metals (e.g. scandium, titanium, vanadium, chromium, manganese, iron, cobalt, nickel, copper, zinc, yttrium, zirconium, niobium, molybdenum, technetium, ruthenium, rhodium, palladium, silver, and cadmium; particularly cobalt, nickel and copper), post-transition metals (e.g. aluminium, gallium, indium, and tin) and/or lanthanide elements (e.g. cerium, praseodymium, neodymium, promethium, samarium, europium, gadolinium, terbium, dysprosium, holmium, erbium, thulium, ytterbium, and lutetium). The dopant material may optionally be present in the material at a concentration of from about 0.001 at% to about 10 at%, e.g. from 0.01 at% to about 1 at% such as about 0.1 at%. Preferred examples of doped carbon materials include carbon doped with boron, nitrogen, cobalt, nickel and/or copper. The carbon in a doped carbon material may be selected from graphite, carbon nanotube(s), carbon black, activated carbon, carbon nanopowder, vitreous carbon, carbon fibre(s), carbon cloth, carbon felt, carbon paper, graphene, highly oriented pyrolytic graphite, pyrolytic graphite, carbon quantum dots, and diamond (optionally surface-modified diamond).

For example, when the carbon material comprises doped graphene, the graphene may preferably be doped with one or more dopants selected from nitrogen, boron, sulphur, oxygen, silicon, phosphorus, lanthanide elements and transition-metals. When the carbon material comprises doped carbon nanotube(s), the carbon nanotube(s) may preferably be doped with one or more dopants selected from nitrogen, boron, sulphur, oxygen, silicon, phosphorus, lanthanide elements and transition-metals. When the carbon material comprises doped diamond, the diamond may preferably be doped with one or more dopants selected from nitrogen, boron, sulphur, oxygen, silicon and phosphorus. When the carbon material comprises doped carbon black, the carbon black may preferably be doped with one or more dopants selected from nitrogen, boron, sulphur, oxygen, silicon, phosphorus, lanthanide elements and transition-metals. When the carbon material comprises doped activated carbon, the activated carbon may preferably be doped with one or more dopants selected from nitrogen, boron, sulphur, oxygen, silicon, phosphorus, lanthanide elements and transition-metals.

Examples of surface-modified carbon materials include carbon materials comprising acidic surface groups (e.g. carboxylic acids, carboxylic acid anhydrides, lactones, hydroxyls); basic surface groups (e.g. amides, imides, lactames, carbonyls, amines, imines, pyrrolic and pyridinic groups), and surface groups such as nitro, diazonium, nitroso, fluoro, chloro, bromo, and iodo groups. Functional groups on the carbon material can be introduced by processes such as oxidation (e.g. using reagents such as using nitric acid, sulfuric acid, hydrogen peroxide, potassium permanganate, air, ozone, plasma, etc.), amidation, silanization, silylation, polymer grafting, polymer wrapping, surfactant adsorption, and encapsulation. Such modifications can be generally applied to carbon materials as disclosed herein. In one embodiment, acid oxidation to produce carboxylic acid groups on the carbon surface is preferred. Thus, in one embodiment preferred support materials include carbon black with carboxylic acid surface groups, activated carbon with carboxylic acid surface groups and carbon nanotube(s) with carboxylic acid surface groups.

Preferably, the or each support is an electronically conducting particle. Preferred electronically conducting particles comprise materials described herein. Preferably, when the or each support comprise particles, the particles have a particle size of from about 1 nm to about 100 µm, such as from about 10 nm to about 10 µm e.g. from about 100 nm to about 1 µm. Processes of determining particle size are routine in the art and include, for example, dynamic light scattering. Suitable electronically conducting particles for use in the processes of the invention include conductive carbon black particles such as "Black Pearls 2000" particles available from Cabot corp (Boston, Mass., USA); activated carbon such as Activated Charcoal "DARCO" (~100 mesh) and Vulcan XC72R.

In one embodiment, the weight ratio of the support material to the catalyst is 40 or more:1. Typically, the weight ratio of the support material to the catalyst is 50 or more:1. More typically, the weight ratio of the support material to the catalyst is 100 or more:1. Without wishing to be bound by theory, such a weight ratio may be preferred for a process of reducing a nitro functional group to an amine.

In one embodiment, the weight ratio of the support material to the catalyst is 39 or less:1. More typically, the weight ratio of the support material to the catalyst is 30 or less:1. More typically, the weight ratio of the support material to the catalyst is 20 or less:1. More typically, the weight ratio of the support material to the catalyst is 10 or less:1. More typically, the weight ratio of the support material to the catalyst is 5 or less:1. More typically, the weight ratio of the support material to the catalyst is 1 or less:1. Without wishing to be bound by theory, such a weight ratio may be preferred for a process of reducing a nitro functional group to a hydroxylamine.

In one embodiment, the weight ratio of the support material to the catalyst is 1:1 to 100:1. Typically, the weight ratio of the support material to the catalyst is 1:1 to 50:1.

Typically, the weight ratio of the support material to the catalyst is 1:1 to 10:1. Typically, the weight ratio of the support material to the catalyst is 30:1 to 50:1.

In one embodiment, the catalyst/support material combination is selected from the group consisting of NiArg/C, NiArg/NCNT, Ni(-CH₂Py)/C and Ni(-CH₂Py)/NCNT. Typically, the catalyst and support material in combination is selected from the group consisting of NiArg/C, NiArg/NCNT and Ni(-CH₂Py)/C. Typically, the catalyst and support material are NiArg/C.

As used herein NCNTs refers to nitrogen doped carbon nanotubes.

In one embodiment, the catalyst may be prepared and then used in the process without being isolated between the preparation and use in the process. Typically, the catalyst may be immobilised on a support material, and then used in the process without being isolated.

### Reductant

In one embodiment, the molecular reductant is H₂. Typically, the H₂ is provided by adding gaseous H₂ or a gas mixture comprising gaseous H₂ into a reaction vessel in which the process is performed; optionally the gaseous H₂ or gas mixture are provided at atmospheric pressure.

When the molecular reductant is hydrogen, suitable isotopes thereof include ¹H₂, ²H₂ and ³H₂. Mixed isotopes (e.g. ¹H²H and ¹H³H) are also embraced. Typically, the hydrogen is ¹H₂.

Typically, when the reductant is H₂, the H₂ is provided in the form of a gas. The gas may be mixed with an aqueous solution in which the catalyst and target compound as well as optionally other reaction components are present. At 1 bar H₂ the solubility of H₂ in water is 0.8 mM, at 25 °C. In other words, when the reductant is H₂ provided in the form of molecular hydrogen gas, the concentration of hydrogen is solution is 0.8 mM hydrogen. Other pressures may also be used. Other temperatures may also be used. For example, the gas pressure in the reaction vessel may be from 0.01 to about 100 bar, such as from 0.1 to 10 bar, e.g. from about 0.2 to about 5 bar, e.g. from 0.5 to 2 bar, such as approximately 1 bar. Increasing the gas pressure will increase the concentration of hydrogen in the reaction solution. Decreasing the gas pressure will decrease the concentration of the hydrogen in the reaction solution.

For avoidance of doubt, the H₂ may also be provided in the form of a solution (e.g. an aqueous solution, e.g. comprising buffer salts as described in more detail here) in which molecular hydrogen is dissolved.

When the reductant is H₂, the hydrogen may be provided as a mixture of hydrogen and other gases such as CO, COz, air, O₂, N₂, Ar, etc. When provided as a mixture, the mixture may comprise from about 0.1% to about 99% hydrogen, such as from 1% to about 95%, e.g. from about 2% to about 80% H₂, such as from about 5% to about 50% H₂. An exemplary mixture for example may comprise 50% hydrogen and 50% N₂.

When the reductant is H₂, the hydrogen may be of any suitable purity. Typically, hydrogen of 80% purity or greater (e.g. 85%, 90%, 95% or 98%) may be used. More typically, hydrogen of 99% purity or greater (e.g. 99.9%, 99.99% or 99.999%) may be used. In other embodiments, lower purity hydrogen may be used.

When the reductant is H₂ or an isotope thereof, the molecular hydrogen or isotope thereof may be provided from any suitable source, such as a gas cylinder. Alternatively, H₂ or an isotope thereof can be produced in situ e.g. by electrolysis of water.

### Target compound

The target compound comprises a nitrogen-containing functional group. Typically, the nitrogen-containing functional group is selected from the group consisting of nitro, hydroxylamine, azido, azo, azoxy and nitroso. Typically, the nitrogen-containing functional group is a nitro functional group or a hydroxylamine functional group. Most typically, the nitrogen-containing functional group is a nitro functional group.

As used herein a target compound comprising a nitro functional group refers to R-NO₂ wherein R represents the remainder of the target compound.

As used herein a target compound comprising a hydroxylamine functional group refers to R-NR'OH, wherein R represents the remainder of the target compound and R' represents a group such as H or hydrocarbyl, e.g, alkyl, e.g. C₁₋₆ or C₁₋₄ alkyl such as methyl or ethyl; typically H.

As used herein a target compound comprising an azido functional group refers to 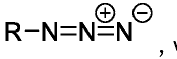 wherein R represents the remainder of the target compound.

As used herein a target compound comprising an azo functional group refers to R-N=N-R', wherein R represents the remainder of the target compound and R' represents a group such as H or hydrocarbyl, e.g, alkyl, e.g. C₁₋₆ or C₁₋₄ alkyl such as methyl or ethyl; typically H.

As used herein a target compound comprising an azoxy functional group refers to 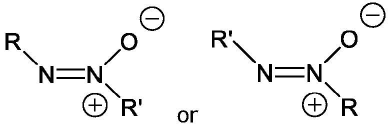 wherein R represents the remainder of the target compound and R' represents a group such as H or hydrocarbyl, e.g, alkyl, e.g. C₁₋₆ or C₁₋₄ alkyl such as methyl or ethyl; typically H. Typically, the azoxy functional group refers to 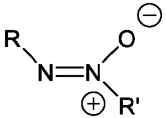 wherein R represents the remainder of the target compound and R' represents a group such as H or hydrocarbyl, e.g, alkyl, e.g. C₁₋₆ or C₁₋₄ alkyl such as methyl or ethyl; typically H.

As used herein a target compound comprising a nitroso group refers to R-N=O, wherein R represents the remainder of the target compound.

The target compound may be any target compound, and selection of appropriate target compounds for use in accordance with the disclosed process is an operational parameter of the disclosed processes and is well within the capacity of those skilled in the art.

Amine synthesis has utility for the production of pharmaceuticals, polyurethanes, and pesticides. Accordingly, in one embodiment, the compound prepared by the process may be a pharmaceutical, polyurethane, pesticide or an intermediate thereof.

Hydroxylamine synthesis has utility for the production of polymerisation inhibitors and precursors in fine chemical synthesis. Accordingly, in one embodiment, the compound prepared by the process may be a polymerisation inhibitor, a precursor in fine chemical synthesis or an intermediate thereof.

The characterization of the products obtained from the processes of the invention is well within the capacity of those skilled in the art. For example, products can be characterized by chemical analytical techniques such as IR spectroscopy, NMR, GC (including chiral-phase GC), polarimetry, mass spectroscopy, HPLC, etc. Exemplary processes are provided in the examples.

The target compound is typically an organic compound and typically has a molecular weight of from about 20 to about 2000 g/mol, such as from about 50 to about 1000 g/mol e.g. from about 100 to about 700 g/mol.

In one embodiment, the nitrogen-containing functional group (such as a nitro functional group or a hydroxylamine functional group) is attached (e.g. covalently attached as a substituent) to an aromatic group comprised in the target compound (R), although non-aromatic groups are also embraced in the processes of the invention. Aromatic groups include hydrocarbyl aromatic groups (such as benzene) and heteroaromatic groups (such as pyridine), which may be further substituted.

In one embodiment, the target compound is a nitroaromatic compound. Typically, the nitroaromatic compound comprises a hydrocarbyl aromatic group or a heteroaromatic group substituted with a nitro group, wherein said hydrocarbyl aromatic group or a heteroaromatic group is optionally further substituted.

In one embodiment, the target compound is an aromatic compound comprising a hydroxylamine functional group. Typically, the aromatic compound comprises a hydrocarbyl aromatic group or a heteroaromatic group substituted with a hydroxylamine group, wherein said hydrocarbyl aromatic group or a heteroaromatic group is optionally further substituted.

In one embodiment, the nitrogen-containing functional group (such as a nitro functional group or a hydroxylamine functional group) is attached (e.g. covalently attached as a substituent) to an aliphatic group. Exemplary aliphatic (non-aromatic) groups include alkyl, alkenyl, cyclic and heterocyclic groups, which may be further substituted.

In one embodiment, the target compound is an aliphatic compound comprising a nitro group. Typically, the target compound is an alkyl compound comprising a nitro group or a cycloalkyl compound comprising a nitro group.

In one embodiment, the target compound is an aliphatic compound comprising a hydroxylamine group. Typically, the target compound is an alkyl compound comprising a hydroxylamine group or a cycloalkyl compound comprising a hydroxylamine group.

Conversion of both aromatic and aliphatic compounds is demonstrated in the examples, demonstrating the broad applicability of the disclosed processes to a wide variety of target compounds.

A target compound may typically comprise a plurality of such groups bonded together, with optional further substitutions.

The substituents that may be present on a target compound (e.g. on a group in a target compound as disclosed herein) are not limited. Substituents are typically selected from the group consisting of F, Br, Cl, I, CN, OR⁵, N(R⁵)₂, -N(R⁵)-C(=NR⁵)-NR⁵₂, SR⁵, SOR⁵, SO₂R⁵, C(O)R⁵, C(O)OR⁵, and C(O)NR⁵₂, C₁ to C₆ (e.g. C₁ to C₃) alkyl groups, C₁ to C₆ (e.g. C₁ to C₃) alkyl groups substituted with 1, 2 or 3 halogens (e.g. F, Br, Cl, I), -CH=CH₂ and -C≡CH; wherein R⁵ is H or C₁ to C₃ alkyl. Typically, selected from the group consisting of F, Br, Cl, I, CN, OR⁵, N(R⁵)₂, SR⁵, SOR⁵, SO₂R⁵, C(O)R⁵, C(O)OR⁵, and C(O)NR⁵₂, C₁ to C₆ (e.g. C₁ to C₃) alkyl groups, C₁ to C₆ (e.g. C₁ to C₃) alkyl groups substituted with 1, 2 or 3 halogens (e.g. F, Br, Cl, I), -CH=CH₂ and -C≡CH; wherein R⁵ is H or C₁ to C₃ alkyl. Typically, selected from the group consisting of F, Br, Cl, I, OR⁵, N(R⁵)₂, SR⁵, SOR⁵, SO₂R⁵, C(O)R⁵, C(O)OR⁵, and C(O)NR⁵₂, C₁ to C₆ (e.g. C₁ to C₃) alkyl groups, C₁ to C₆ (e.g. C₁ to C₃) alkyl groups substituted with 1, 2 or 3 halogens (e.g. F, Br, Cl, I), -CH=CH₂ and -C≡CH; wherein R⁵ is H or C₁ to C₃ alkyl. More typically, substituents are selected from the group consisting of F, Br, Cl, I, C₁ to C₆ (e.g. C₁ to C₃) alkyl groups, -CH=CH₂ and -C≡CH;, wherein R⁵ is H or C₁ to C₃ alkyl. Most typically, substituents are selected from the group consisting of F, Br, Cl, I, C₁ to C₆ (e.g. C₁ to C₃) alkyl groups, wherein R⁵ is H or C₁ to C₃ alkyl. Typically, a substituted group comprises 1, 2, 3 or 4 substituents, more typically 1, 2 or 3 substituents, more typically 1 or 2 substituents, and more typically 1 substituent. For the avoidance of doubt, in a target compound, any substituent is in addition to any nitro functional groups or hydroxylamine functional groups.

As used herein, R may comprise or consist of any suitable aromatic or aliphatic group or combination of groups. Suitable groups are defined herein.

In one embodiment, R is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton. Typically, R is a saturated or unsaturated C₁-C₂₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton. Typically, the hydrocarbyl includes up to one heteroatom in its carbon skeleton. More typically, the hydrocarbyl includes no heteroatoms in its carbon skeleton. More typically, the hydrocarbyl includes 0, 1 or 2 substituents. More typically, R is a C₁₋₁₀ alkyl group, C₁₋₁₀ alkenyl group, C₁₋₁₀ alkynyl group, or a 5-6 membered cyclic group; wherein the group may be optionally substituted, and wherein the group may optionally include one or two heteroatoms N, O or S in its carbon skeleton. More typically, R is a C₁₋₁₀ alkyl group, or a 5-6 membered cyclic group; wherein the group may be optionally substituted, and wherein the group may optionally include one or two heteroatoms N, O or S in its carbon skeleton.

For avoidance of doubt, a plurality of nitrogen-containing functional groups (such as nitro functional groups and/or hydroxylamine functional groups) may be comprised in the target compound and may be reduced according to the processes disclosed herein. Typically, the target compound comprises one nitrogen-containing functional group.

Typically, the target compound comprises one nitro functional group or one hydroxylamine functional group. Typically, the target compound comprises one or more (e.g. 1, 2, 3 or 4) nitro functional groups. Typically, the target compound comprises two or more nitro functional groups. Typically, the target compound comprises one or two nitro functional groups. Typically, the target compound comprises two nitro functional groups. Typically, the target compound comprises two nitro functional groups, a first and second nitro group respectively, and only a first nitro group is reduced. More typically, the target compound comprises two nitro functional groups and both are reduced. Most typically, the target compound comprises one nitro functional group.

In one embodiment, when the process is a process of reducing two nitro functional groups in a target compound, and the product is an amine, the catalyst may be used in heterogeneous form supported on a support material, with weight ratio of the support material to the catalyst of 40 or more:1. Where the target molecule comprises two nitro functional groups, a first and a second nitro group respectively, the process conditions may be adjusted to reduce one preferentially to the other. Typically, where the target compound comprises a first and a second nitro group at least 50 mol%, e.g. at least 60 mol%, at least 70 mol%, at least 80 mol%, at least 90 mol%, at least 95 mol%, at least 97 mol%, at least 98 mol%, at least 99 mol %, at least 99.9 mol % or at least 99.99 mol % of the first nitro functional group in the target compounds in the sample may be completely reduced to an amine. The second nitro group may or may not be reduced. Typically, the second nitro group is not reduced. More typically, the second nitro group is reduced.

Typically, when the process is a process of reducing two nitro functional groups in a target compound, and the product is a hydroxylamine, the catalyst may be used in homogeneous form or in heterogeneous form on a support material with a weight ratio of the support material to the catalyst of 39 or less:1. Where the target molecule comprises two nitro functional groups, a first and a second nitro group respectively, the process conditions may be adjusted to reduce one preferentially to the other. Typically, with a weight ratio of the support material to the catalyst of 30 or less: 1. More typically, with a weight ratio of the support material to the catalyst of 10 or less: 1. Typically, at least 50 mol%, e.g. at least 60 mol%, at least 70 mol%, at least 80 mol%, at least 90 mol%, at least 95 mol%, at least 97 mol%, at least 98 mol%, at least 99 mol %, at least 99.9 mol % or at least 99.99 mol % of the first nitro functional group in the target compounds in the sample may be completely reduced to a hydroxylamine. The second nitro group may or may not be reduced. Typically, the second nitro group is not reduced. More typically, the second nitro group is reduced.

In one embodiment, the nitrogen-containing functional group (such as a nitro functional group or hydroxylamine functional group) is attached to a non-saturated carbon atom.

In one embodiment, the nitrogen-containing functional group (such as a nitro functional group or a hydroxylamine functional group) is attached to an aromatic (hydrocarbyl aromatic or heteroaromatic) group. More typically the target compound comprises an aromatic nitro group. In one embodiment the nitrogen-containing functional group (such as a nitro functional group or a hydroxylamine functional group) is covalently attached to an aromatic group which is further *para* substituted relative to the nitro functional group or hydroxylamine functional group. In one embodiment the nitrogen-containing functional group (such as a nitro functional group or a hydroxylamine functional group) is covalently attached to an aromatic group which is further *meta* substituted relative to the nitro functional group or hydroxylamine functional group. In one embodiment the nitrogen-containing functional group (such as a nitro functional group or hydroxylamine functional group) is covalently attached to an aromatic group which is *ortho* substituted relative to the nitro functional group or hydroxylamine functional group.

In one embodiment the target compound comprises multiple functional groups, for example the target compound may comprise a reducible nitro functional group or hydroxylamine functional group and a functional group such as a C=C double bond. Typically, the processes provided herein are capable of selectively reducing the nitro functional group or hydroxylamine functional group without reducing the non-nitrogenous functional group.

As will thus be apparent, the disclosed processes comprise the formation of a reaction product comprising a reduced functional group, e.g. comprising a hydroxylamine and/or an amine when the target compound comprises a nitro functional group; or comprising an amine when the target compound comprises a hydroxylamine functional group. Typically, the disclosed processes comprise the formation of a reaction product comprising an amine.

### Hydrocarbyl groups

In the context of the present specification, a "hydrocarbyl" group includes carbon and hydrogen atoms but, unless stated otherwise, does not include any heteroatoms, such as N, O or S, in its carbon skeleton. A hydrocarbyl group may be saturated or unsaturated (including aromatic), and may be straight-chained or branched, or be or include cyclic groups wherein, unless stated otherwise, the cyclic group does not include any heteroatoms, such as N, O or S, in its carbon skeleton. Examples of hydrocarbyl groups include alkyl, alkenyl, alkynyl, cycloalkyl, cycloalkenyl and aryl groups/moieties and combinations of all of these groups/moieties. Typically, a hydrocarbyl group is a C₁ to C₃₀ hydrocarbyl group. Typically, a hydrocarbyl group is a C₁ to C₂₀ hydrocarbyl group. More typically a hydrocarbyl group is a C₁ to C₁₅ hydrocarbyl group. More typically a hydrocarbyl group is a C₁ to C₁₀ hydrocarbyl group. A "hydrocarbylene" group is similarly defined as a divalent hydrocarbyl group.

In the context of the present specification, unless otherwise stated, an "alkyl" substituent group or an alkyl moiety in a substituent group may be linear or branched. Examples of alkyl groups/moieties include methyl, ethyl, n-propyl, iso-propyl, n-butyl, iso- butyl, tert-butyl and n-pentyl groups/moieties. Unless stated otherwise, the term "alkyl" does not include "cycloalkyl". Typically, an alkyl group is a C₁-C₂₀ alkyl group. More typically, an alkyl group is a C₁-C₁₂ alkyl group. More typically an alkyl group is a C₁-C₆ alkyl group. An "alkylene" group is similarly defined as a divalent alkyl group.

An "alkenyl" substituent group or an alkenyl moiety in a substituent group refers to an unsaturated alkyl group or moiety having one or more carbon-carbon double bonds. Examples of alkenyl groups/moieties include ethenyl, propenyl, l-butenyl, 2-butenyl, l-pentenyl, l-hexenyl, 1,3-butadienyl, 1,3-pentadienyl, 1,4-pentadienyl and 1,4-hexadienyl groups/moieties. Unless stated otherwise, the term "alkenyl" does not include "cycloalkenyl". Typically, an alkenyl group is a C₂-C₂₀ alkenyl group. Typically, an alkenyl group is a C₂-C₁₂ alkenyl group. More typically an alkenyl group is a C₂ -C₆ alkenyl group. An "alkenylene" group is similarly defined as a divalent alkenyl group.

An "alkynyl" substituent group or an alkynyl moiety in a substituent group refers to an unsaturated alkyl group or moiety having one or more carbon-carbon triple bonds. Examples of alkynyl groups/moieties include ethynyl, propargyl, but-1-ynyl and but-2-ynyl. Typically, an alkynyl group is a C₂ -C₂₀ alkynyl group. Typically, an alkynyl group is a C₂ -C₆ alkynyl group. An "alkynylene" group is similarly defined as a divalent alkynyl group.

A "cyclic" substituent group or a cyclic moiety in a substituent group refers to any hydrocarbyl ring, wherein the hydrocarbyl ring may be saturated or unsaturated (including aromatic) and may include one or more heteroatoms, e.g. N, O or S, in its carbon skeleton. Examples of cyclic groups include cycloalkyl, cycloalkenyl, heterocyclic, aryl and heteroaryl groups as discussed below. A cyclic group may be monocyclic, bicyclic (e.g. bridged, fused or spiro), or polycyclic. Typically, a cyclic group is a 3- to 12-membered cyclic group, which means it contains from 3 to 12 ring atoms. More typically, a cyclic group is a 3- to 7-membered monocyclic group, which means it contains from 3 to 7 ring atoms.

As used herein, where it is stated that a cyclic group is monocyclic, it is to be understood that the cyclic group is not substituted with a divalent bridging substituent (e.g. -0-, -S-, -NH-) so as to form a bridged, fused or spiro substituent. However, unless stated otherwise, a substituted monocyclic group may be substituted with one or more monovalent cyclic groups. Similarly, where it is stated that a group is bicyclic, it is to be understood that the cyclic group including any bridged, fused or spiro divalent bridging substituents attached to the cyclic group, but excluding any monovalent cyclic substituents, is bicyclic.

A "heterocyclic" substituent group or a heterocyclic moiety in a substituent group refers to a cyclic group or moiety including one or more carbon atoms and one or more heteroatoms, e.g. N, O or S, in the ring structure. Examples of heterocyclic groups include heteroaryl groups as discussed below and non-aromatic heterocyclic groups. Examples of heterocyclic groups include piperazine, piperidine, morpholine, 1,3-oxazinane, pyrrolidine, imidazolidine, oxazolidine; tetrahydropyrazine, tetrahydropyridine, dihydro-1,4-oxazine, tetrahydropyrimidine, dihydro-1,3-oxazine, dihydropyrrole, dihydroimidazole, dihydrooxazole, indoline, 2,3-dihydrobenzofuran, 2,3-dihydrobenzo[b]thiophene, 2,3-dihydro-1H-benzo[d]imidazole, 2,3-dihydrobenzo[d]oxazole, 2,3-dihydrobenzo[d]thiazole, benzo[d][1,3]dioxole, 4,5,6,7-tetrahydrothiazolo[5,4-c]pyridine and 4,5,6,7-tetrahydrothiazolo[4,5-c]pyridine, 1,2,3,4-tetrahydroquinoline, 1,2,3,4-tetrahydroisoquinoline, chromane, isochromane, thiochromane, isothiochromane, 1,2,3,4-tetrahydroquinoxaline, 1,2,3,4-tetrahydroquinazoline, 1,4-dihydro-2H-benzo[d][1,3]oxazine, 3,4-dihydro-2H-benzo[b][1,4]oxazine, 3,4-dihydro-2H-benzo[b][1,4]thiazine, 1,4-dihydro-2H-benzo[d][1,3]thiazine, 4H-benzo[d][1,3]dioxine and 2,3-dihydrobenzo[b][1,4]dioxine, including quaternised derivatives thereof.

A "cycloalkyl" substituent group or a cycloalkyl moiety in a substituent group refers to a saturated hydrocarbyl ring containing, for example, from 3 to 7 carbon atoms, examples of which include cyclopropyl, cyclobutyl, cyclopentyl and cyclohexyl. Unless stated otherwise, a cycloalkyl substituent group or moiety may include monocyclic, bicyclic or polycyclic hydrocarbyl rings.

A "cycloalkenyl" substituent group or a cycloalkenyl moiety in a substituent group refers to a non-aromatic unsaturated hydrocarbyl ring having one or more carbon- carbon double bonds and containing, for example, from 3 to 7 carbon atoms, examples of which include cyclopent-1-en-1-yl, cyclohex-1-en-1-yl and cyclohex-1,3-dien-1-yl. Unless stated otherwise, a cycloalkenyl substituent group or moiety may include monocyclic, bicyclic or polycyclic hydrocarbyl rings.

An "aryl" substituent group or an aryl moiety in a substituent group refers to an aromatic hydrocarbyl ring. The term "aryl" includes monocyclic aromatic hydrocarbons and polycyclic fused ring aromatic hydrocarbons wherein all of the fused ring systems (excluding any ring systems which are part of or formed by optional substituents) are aromatic. Examples of aryl groups/moieties include phenyl, naphthyl, anthracenyl and phenanthrenyl. Unless stated otherwise, the term "aryl" does not include "heteroaryl".

A "heteroaryl" substituent group or a heteroaryl moiety in a substituent group refers to an aromatic heterocyclic group or moiety. The term "heteroaryl" includes monocyclic aromatic heterocycles and polycyclic fused ring aromatic heterocycles wherein all of the fused ring systems (excluding any ring systems which are part of or formed by optional substituents) are aromatic. A heteroaromatic group is typically a 5- to 10-membered heteroaryl group, which is a substituted or unsubstituted monocyclic or fused polycyclic aromatic group containing from 5 to 10 atoms in the ring portion, including at least one heteroatom, for example 1, 2 or 3 heteroatoms, typically selected from O, S and N. Examples of heteroaryl groups include pyrrole, furan, thiophene, imidazole, oxazole, thiazole, pyridine, pyridazine, pyrimidine, pyrazine, indole, benzothiophene, benzofuran, benzoxazole, benzothiazole, benzimidazole, imidazo[1,2-a]pyridine, [1,2,4]triazolo[1,5-a]pyridine, imidazo[1,2-a]pyrazine, quinoline, isoquinoline, quinazoline, and quinoxaline. A heteroaryl group may be a fused polycyclic ring systems, including for instance fused bicyclic systems in which a heteroaryl group is fused to an aryl group as defined herein.

For the purposes of the present specification, where a combination of moieties is referred to as one group, for example, alkylaryl, the first mentioned moiety contains the atom by which the group is attached to the rest of the molecule. An example of an alkylaryl group is benzyl or -CH₂-pyrenyl.

Substituents are typically selected from the group consisting of F, Br, Cl, I, CN, OR⁵, N(R⁵)₂, -N(R⁵)-C(=NR⁵)-NR⁵₂, SR⁵, SOR⁵, SO₂R⁵, C(O)R⁵, C(O)OR⁵, and C(O)NR⁵₂, C₁ to C₆ (e.g. C₁ to C₃) alkyl groups, C₁ to C₆ (e.g. C₁ to C₃) alkyl groups substituted with 1, 2 or 3 halogens (e.g. F, Br, Cl, I), -CH=CH₂ and -C≡CH; wherein R⁵ is H or C₁ to C₃ alkyl. More typically, substituents are selected from the group consisting of F, Br, Cl, I, -N(R⁵)-C(=NR⁵)-NR⁵₂, C₁ to C₆ (e.g. C₁ to C₃) alkyl groups, -CH=CH₂ and -C≡CH; wherein R⁵ is H or C₁ to C₃ alkyl. Most typically, substituents are selected from the group consisting of F, Br, Cl, I, C₁ to C₆ (e.g. C₁ to C₃) alkyl groups, wherein R⁵ is H or C₁ to C₃ alkyl. Typically, a substituted group comprises 1, 2, 3 or 4 substituents, more typically 1, 2 or 3 substituents, more typically 1 or 2 substituents, and more typically 1 substituent.

Where reference is made to a hydrocarbyl or other group including one or more heteroatoms N, O or S in its carbon skeleton, or where reference is made to a carbon atom of a hydrocarbyl or other group being replaced by an N, O or S atom, what is intended is that: 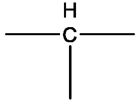 is replaced by 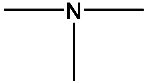
-CH₂ - is replaced by -NH-, -O- or -S-;
-CH₃ is replaced by -NH₂, -OH, or -SH;
-CH= is replaced by -N=;
CH₂ = is replaced by NH=, 0= or S=; or
CHz is replaced by N≡;
provided that the resultant group comprises at least one carbon atom.

### Reaction conditions

In one embodiment, the process comprises reducing a nitro functional group to form a hydroxylamine.

In one embodiment, the process comprises reducing a nitro functional group to form an amine.

In one embodiment, the process comprises reducing a hydroxylamine functional group to form an amine.

In one embodiment, the process comprises reducing an azido functional group to form an amine.

In one embodiment, the process comprises reducing an azo functional group to form an amine.

In one embodiment, the process comprises reducing an azoxy functional group to form an amine.

In one embodiment, the process comprises reducing a nitroso functional group to form an amine.

In the disclosed processes, the target compound is preferably initially added to or present in the reaction medium at a concentration of 1 µM to 1 M, such as from 5 µM to 800 mM, e.g. from 10 µM to 600 mM such as from 25 µM to 400 mM e.g. from 50 µM to 200 mM such as from 100 µM to about 100 mM e.g. from about 250 µM to about 10 mM such as from about 500 µM to about 1 mM.

As explained above, when the molecular reductant is a gas such as hydrogen, the disclosed processes are typically conducted under a gas atmosphere; i.e. in the presence of gas (for example in the headspace of a reactor). Preferably, the gas atmosphere comprises hydrogen or an isotope thereof and optionally an inert gas. O₂ or an isotope thereof may be present. Preferred inert gases include nitrogen, argon, helium, neon, krypton, xenon, radon and sulfur hexafluoride (SF₆) and mixtures thereof, more preferably nitrogen and/or argon, most preferably nitrogen.

When the gas atmosphere comprises a mixture of hydrogen and an inert gas and/or O₂, the hydrogen is preferably present at a concentration of 1-100%, with the remaining gas comprising an inert gas as defined herein and/or O₂. Preferred gas atmospheres include from 80-100% H₂ with the remaining gas comprising one or more inert gases; and from 0-20% H₂ with the remaining gas comprising one or more inert gases and/or O₂ (such as from 1-4% H₂ in air).

The gas atmosphere may optionally also include non-inert gases such as ammonia, carbon dioxide and hydrogen sulphide. Preferably, however, the gas atmosphere is free of ammonia, carbon dioxide and hydrogen sulphide.

The processes of the invention may be conducted at any suitable pressure: selecting an appropriate pressure is an operational parameter of the processes of the invention which can be controlled by the operator. Sometimes, the processes of the invention are conducted at ambient pressure (e.g. about 1 bar). Sometimes, the processes of the invention are conducted at reduced pressure (e.g. less than 1 bar) or at elevated pressure (e.g. greater than 1 bar). For example, increasing the operating pressure can increase hydrogen solubility in the reaction medium. Preferably, the processes of the invention are carried out at a pressure of from about 0.1 bar to about 20 bar, such as from about 1 bar to about 10 bar, e.g. from about 2 bar to about 8 bar such as from about 4 bar to about 6 bar, e.g. about 5 bar.

The disclosed processes may be carried out under aerobic or anaerobic conditions. As used herein, "aerobic conditions" refers to the gas atmosphere not being strictly anaerobic, e.g. comprising at least trace O₂. Suitable O₂ levels are typically greater than 100 ppm, e.g. greater than 1000 ppm (0.1%), such as greater than 1 % O₂, for example greater than 2% O₂. Usually, O₂ levels do not exceed the O₂ levels in atmospheric air, i.e. 21% Oz, however greater O₂ levels are not excluded. Typically, the process is performed anaerobically.

In one embodiment, the process is performed in the presence of a protic solvent. Typically, the process is performed in the presence of a protic solvent, wherein the protic solvent comprises water or aqueous sodium phosphate.

In one embodiment, the process is performed in the presence of a solvent comprising water and optionally one or more water miscible organic solvent. Examples of water miscible organic solvents include acetonitrile, acetone, alcohols and DMSO. Typically, the process is performed in the presence of a solvent comprising water and acetonitrile. More typically, the process is performed in the presence of a solvent comprising water.

The provided processes are typically conducted in the presence of a solvent comprising water optionally comprising buffer salts.

The provided processes are typically conducted in a solvent comprising water and which may optionally comprise e.g. buffer salts. Preferred buffer salts which can be used in the processes of the invention include Tris; phosphate; citric acid / Na₂HPO₄; citric acid / sodium citrate; sodium acetate / acetic acid; Na₂HPO₄ / NaH₂PO₄; imidazole (glyoxaline) / HCl; sodium carbonate / sodium bicarbonate; ammonium carbonate / ammonium bicarbonate; MES; Bis-Tris; ADA; aces; PIPES; MOPSO; Bis-Tris Propane; BES; MOPS; TES; HEPES; DIPSO; MOBS; TAPSO; Trizma; HEPPSO; POPSO; TEA; EPPS; Tricine; Gly-Gly; Bicine; HEPBS; TAPS; AMPD; TABS; AMPSO; CHES; CAPSO; AMP; CAPS and CABS. Selection of appropriate buffers for a desired pH is routine to those skilled in the art, and guidance is available at e.g. http://www.sigmaaldrich.com/life-science/core-bioreagents/biological-buffers/learning-center/buffer-reference-center.html. Buffer salts are preferably used at concentrations of from 1 mM to 1 M, preferably from 10 mM to 100 mM such as about 50 mM in solution. Most preferred buffers for use in processes of the invention include 50 mM phosphate, pH 4-6.

The disclosed processes may be carried out in a mixed solvent system comprising a plurality (e.g. 2, 3, 4, 5 or more) of aqueous or non-aqueous solvents, e.g. solvents as described herein. In one embodiment the disclosed processes are carried out in a monophasic solvent system. In one embodiment the disclosed processes are carried out in a non-miscible solvent mixture such as a biphasic or triphasic solvent system.

The disclosed processes are typically carried out at a temperature of from about 10 °C to about 80 °, such as from about 20 °C to about 60 °C, e.g. from about 20 °C to about 30 °C.

The disclosed processes are typically carried out for 12 hours to 4 days. More typically 18 hours to 3 days. Most typically 18 hours to 30 hours.

The disclosed processes are typically carried out at a pH of from about pH 4 to about pH 10 such as from about pH 5 to about pH 9 e.g. from about pH 5 to about pH 7 e.g. about pH 6.

In one embodiment, the catalyst loading is at least 0.2 mol% catalyst:target compound. Typically, the catalyst loading is at least 0.3 mol% catalyst:target compound. Typically, the catalyst loading is at least 0.4 mol% catalyst:target compound. Typically, the catalyst loading is at least 0.5 mol% catalyst:target compound. Typically, the catalyst loading is at least 1.0 mol% catalyst:target compound.

In one embodiment the catalyst loading is less than 1.0 mol% catalyst:target compound.

Typically, the catalyst loading is less than 0.5 mol% catalyst:target compound.

Typically, the catalyst loading is less than 0.4 mol% catalyst:target compound.

Typically, the catalyst loading is less than 0.3 mol% catalyst:target compound.

Typically, the catalyst loading is less than 0.2 mol% catalyst:target compound.

In one embodiment the catalyst loading is from 0.2-1.0 mol% catalyst:target compound. Typically, the catalyst loading is from 0.2-0.5 mol% catalyst:target compound.

The product provided by the process (e.g. an amine or a hydroxylamine) may optionally be isolated and/or purified by any conventional means.

The product provided by the process may optionally be obtained in the form of a salt. For example, where the product is an amine, the amine may be isolated in the form of a quaternary ammonium salt.

In one embodiment, at least 50 mol% of the target compound molecules are reduced at the functional group to be reduced (e.g. the nitro functional group and/or hydroxylamine functional group). Typically, at least 60 mol%, at least 70 mol%, at least 80 mol%, at least 90 mol%, at least 95 mol%, at least 97 mol%, at least 98 mol%, at least 99 mol%, at least 99.9 mol% or at least 99.99 mol% of the target compound molecules in the sample are reduced at the functional group to be reduced.

In one embodiment, when the target compound comprises a nitro functional group at least 50 mol% of the target compound molecules are reduced to an amine. Typically, at least 60 mol% such as at least 70 mol%, at least 80 mol%, at least 90 mol%, at least 95 mol%, at least 97 mol%, at least 98 mol%, at least 99 mol%, at least 99.9 mol% or at least 99.99 mol% of the target compound molecules in the sample are reduced to an amine. Optionally, the remaining target compound molecules may be reduced to a hydroxylamine.

In one embodiment, when the target compound comprises a nitro functional group at least 50 mol% of the target compound molecules are reduced to a hydroxylamine. Typically, at least 60 mol% such as at least 70 mol%, at least 80 mol%, at least 90 mol%, at least 95 mol%, at least 97 mol%, at least 98 mol%, at least 99 mol%, at least 99.9 mol% or at least 99.99 mol% of the target compound molecules in the sample are reduced to a hydroxylamine. Optionally, the remaining target compound molecules may be reduced to an amine.

In one embodiment, when the target compound comprises a nitro functional group, the molar ratio of product obtained as amine : hydroxylamine is 99:1 to 1:99. Typically, 95:5 to 5:95. Typically, 90:10 to 10:90. Typically 99:1 to 90:10. Typically, 1:99 to 10:90.

The disclosed processes may be performed in an apparatus as described herein. The apparatus typically comprises a reaction vessel. The reaction vessel typically comprises one or more inlets for molecular hydrogen gas or hydrogen-containing liquids (e.g. hydrogen saturated liquids such as buffer solutions as described herein); and/or one or more inlet for reagents; and/or one or more outlets for product. Further equipment such a pressure controls, temperature controls, mixing apparatus, flow controls, etc may be incorporated. The apparatus may be comprised as a part of an apparatus for converting initial reagents into final products and thus be configured to perform an intermediate reaction step. The apparatus may be controlled by equipment such as a computer controller. The apparatus may comprise means for detecting cofactor turnover, reagent utilisation and/or product production, e.g. spectrophotometric means. The apparatus may be configured to be operated in flow mode (i.e. continuous mode) or in batch mode.

Accordingly, the processes provided herein may be performed in a flow setup, e.g. in a flow reaction cell. The processes provided herein may alternatively be performed in a batch setup e.g. in a batch reaction cell.

### System

A second aspect provides a system comprising:
(i) a catalyst of Formula (I) 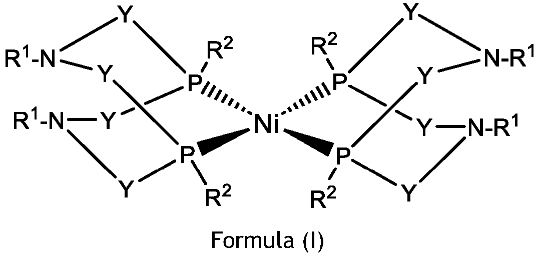 wherein:
   Y, each independently, is C(R³)_{2;} and R³, each independently, is H or C₁₋₃ alkyl;
   R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
   R², each independently, is a 5-6 membered cyclic group, wherein the cyclic group may be optionally substituted, and wherein the cyclic group may optionally include one or more heteroatoms N, O or S in its carbon skeleton;
(ii) a molecular reductant; and
(iii) a target compound comprising a nitrogen-containing functional group;
wherein the system is configured such that, in use, the nitrogen-containing functional group is reduced.

In the systems provided herein, the catalyst is typically as defined herein. The support material is typically as defined herein. The molecular reductant is typically as defined herein. The target compound and functional group comprised therein is typically as defined herein. The system may be configured to be operated as described for the processes provided herein. The system may be configured to be operated under reaction conditions as defined herein.

Typically, the system may further comprise means for controlling a concentration of molecular reductant present in the reaction medium (e.g. by controlling the gas atmosphere in the system, e.g. by using a gas flow system). The system is often configured as a flow cell containing reagents as described herein. The system (e.g. a flow cell) may comprise features of the provided apparatus described herein, such as one or more inlets for the molecular reductant (e.g. hydrogen) and/or one or more inlet for reagents; and/or one or more outlets for product; and/or one or more pressure controls, temperature controls, mixing apparatus, flow controls, etc.

### Preferred aspects

A preferred embodiment of the first aspect provides a process of reducing a nitro functional group in a target compound, comprising contacting the target compound with a catalyst of Formula (I) in the presence of a molecular reductant: 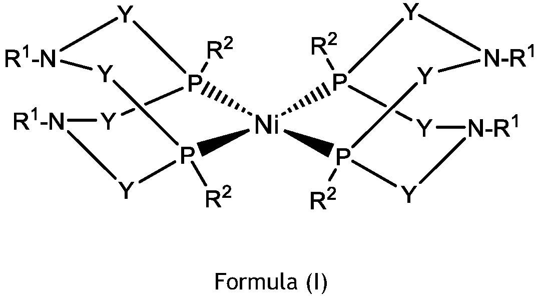 wherein:
Y, each independently, is -C(R³)₂-; and R³, each independently, is H or C₁₋₃ alkyl;
R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
R², each independently, is a 5-6 membered cyclic group, wherein the cyclic group may be optionally substituted, and wherein the cyclic group may optionally include one or more heteroatoms N, O or S in its carbon skeleton.

In one embodiment, the molecular reductant is H₂.

In one embodiment, the process comprises reducing the nitro functional group to form an amine. Typically, the catalyst is used in heterogeneous form. Typically, the catalyst is supported on a support material. More typically, the catalyst is used in heterogeneous form; and the catalyst is supported on a support material; wherein the weight ratio of the support material to the catalyst is 40 or more:1. More typically, the catalyst is used in heterogeneous form; and the catalyst is supported on a support material; wherein the weight ratio of the support material to the catalyst is 50 or more:1. More typically, the catalyst is used in heterogeneous form; and the catalyst is supported on a support material; wherein the weight ratio of the support material to the catalyst is 100 or more:1. Typically, the catalyst loading is at least 0.2 mol% catalyst:target compound. Typically, the catalyst loading is at least 0.3 mol% catalyst:target compound. Typically, the catalyst loading is at least 0.4 mol% catalyst:target compound. Typically, at least 50%, e.g. at least 60%, at least 70%, at least 80%, at least 90%, at least 95%, at least 97%, at least 98%, at least 99%, at least 99.9% or at least 99.99% of the target compound molecules in the sample may be completely reduced to form an amine. More typically, at least at least 95% of the target compound molecules in the sample may be completely reduced to form an amine. Most typically, at least at least 99% of the target compound molecules in the sample may be completely reduced to form an amine.

In one embodiment, the process comprises reducing the nitro functional group to form a hydroxylamine. Typically, the catalyst is used in homogeneous form. Typically, the catalyst loading is at least 0.2 mol% catalyst:target compound. Typically, the catalyst loading is at least 0.3 mol% catalyst:target compound. Typically, the catalyst loading is at least 0.4 mol% catalyst:target compound. Typically, at least 50%, e.g. at least 60%, at

least 70%, at least 80%, at least 90%, at least 95%, at least 97%, at least 98%, at least 99%, at least 99.9% or at least 99.99% of the target compound molecules in the sample may be completely reduced to form a hydroxylamine. More typically, at least at least 95% of the target compound molecules in the sample may be completely reduced to form a hydroxylamine. Most typically, at least at least 99% of the target compound molecules in the sample may be completely reduced to form a hydroxylamine.

In one embodiment, the process comprises reducing the nitro functional group to form a hydroxylamine. Typically, the catalyst is used in heterogeneous form. Typically, the catalyst is supported on a support material. More typically, the catalyst is supported on a support material; wherein the weight ratio of the support material to the catalyst is 30 or less:1. More typically, the catalyst is supported on a support material; wherein the weight ratio of the support material to the catalyst is 20 or less:1. More typically, the catalyst is supported on a support material; wherein the weight ratio of the support material to the catalyst is 10 or less: 1 .Typically, the catalyst loading is at least 0.2 mol% catalyst:target compound. Typically, the catalyst loading is at least 0.3 mol% catalyst:target compound. Typically, the catalyst loading is at least 0.4 mol% catalyst:target compound. Typically, at least 50%, e.g. at least 60%, at least 70%, at least 80%, at least 90%, at least 95%, at least 97%, at least 98%, at least 99%, at least 99.9% or at least 99.99% of the target compound molecules in the sample may be completely reduced to form a hydroxylamine. More typically, at least at least 95% of the target compound molecules in the sample may be completely reduced to form a hydroxylamine. Most typically, at least at least 99% of the target compound molecules in the sample may be completely reduced to form a hydroxylamine.

A preferred embodiment of the second aspect provides a system comprising:
(i) a catalyst of Formula (I) 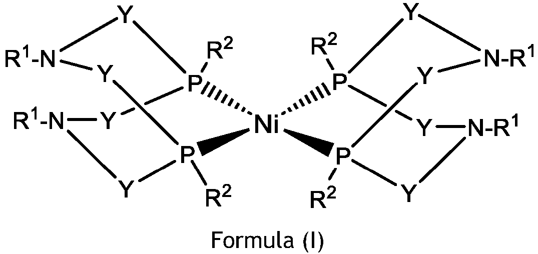 wherein:
   Y, each independently, is C(R³)_{2;} and R³, each independently, is H or C₁₋₃ alkyl;
   R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
   R², each independently, is a 5-6 membered cyclic group, wherein the cyclic group may be optionally substituted, and wherein the cyclic group may optionally include one or more heteroatoms N, O or S in its carbon skeleton;
(ii) a molecular reductant; and
(iii) a target compound comprising a nitro functional group;
wherein the system is configured such that, in use, the nitro functional group is reduced.

### Clauses

Clause 1. A process of reducing a nitrogen-containing functional group in a target compound, comprising contacting the target compound with a catalyst of Formula (I) in the presence of a molecular reductant: 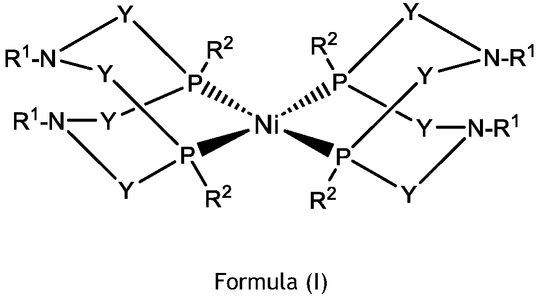 wherein:
Y, each independently, is -C(R³)₂-; and R³, each independently, is H or C₁₋₃ alkyl;
R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
R², each independently, is a 5-6 membered cyclic group, wherein the cyclic group may be optionally substituted, and wherein the cyclic group may optionally include one or more heteroatoms N, O or S in its carbon skeleton.

Clause 2. The process of clause 1, wherein the nitrogen-containing functional group is selected from the group consisting of nitro, hydroxylamine, azido, azo, azoxy and nitroso; optionally wherein the nitrogen-containing functional group is a nitro group or a hydroxylamine functional group.

Clause 3. The process of clause 1 or clause 2, wherein the nitrogen-containing functional group is a nitro functional group.

Clause 4. The process of preceding clause, wherein the process is performed in the presence of a protic solvent; optionally wherein the protic solvent comprises water or aqueous sodium phosphate.

Clause 5. The process of any one of clauses 1-4, wherein the catalyst is used in homogeneous form.

Clause 6. The process of any one of clauses 1-4, wherein the catalyst is used in heterogeneous form; and the catalyst is supported on a support material and the weight ratio of the support material to the catalyst is 10 or less:1.

Clause 7. The process of any one of clauses 1-6, wherein the nitrogen-containing functional group is a nitro functional group, comprising reducing the nitro functional group to form a hydroxylamine.

Clause 8. The process of any one of clauses 1-4, wherein the catalyst is used in heterogeneous form.

Clause 9. The process of clause 8, wherein the catalyst is supported on a support material; and the weight ratio of the support material to the catalyst is 40 or more:1.

Clause 10. The process of clause 6 or clause 9, wherein the support material is electronically conductive or semi-conductive.

Clause 11. The process of clause 10, wherein the support material comprises a carbon material; optionally wherein the carbon material comprises graphite, carbon nanotube(s), carbon black, activated carbon, carbon nanopowder, vitreous carbon, carbon fibre(s), carbon cloth, carbon felt, carbon paper, graphene, highly oriented pyrolytic graphite, pyrolytic graphite, carbon quantum dots, doped or surface-modified carbon or doped diamond.

Clause 12. The process of clause 11, wherein the carbon material comprises carbon black or nitrogen-doped carbon nanotubes.

Clause 13. The process of any one of clauses 6 or 9-12, wherein the catalyst is adsorbed to the support material.

Clause 14. The process of any one of clauses 9-13, wherein the nitrogen-containing functional group is a nitro functional group, comprising reducing the nitro functional group to form an amine.

Clause 15. The process of any preceding clause, wherein the molecular reductant is H₂.

Clause 16. The process of clause 15, wherein the H₂ is provided by adding gaseous H₂ or a gas mixture comprising gaseous H₂ into a reaction vessel in which the process is performed; optionally wherein the gaseous H₂ or gas mixture are provided at atmospheric pressure.

Clause 17. The process of any preceding clause, wherein the target compound comprises two nitro functional groups.

Clause 18. The process of any one of claims 1-16, wherein the target compound comprises one nitro functional group.

Clause 19. The process of any preceding clause, wherein the target compound is a nitroaromatic compound.

Clause 20. The process of any one of clauses 1-18, wherein the target compound is an aliphatic compound comprising a nitro group; optionally wherein the target compound is an alkyl compound comprising a nitro group or a cycloalkyl compound comprising a nitro group.

Clause 21. The process of any preceding clause, wherein at least one R¹ is a group of Formula (II): 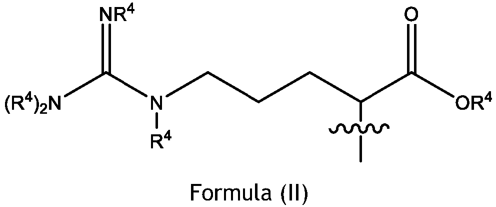 wherein R⁴, each independently, is H or C₁₋₃ alkyl; optionally wherein each R⁴ is H.

Clause 22. The process of any preceding clause, wherein at least one R¹ comprises a guanidino group; optionally wherein at least one R¹ is argininyl.

Clause 23. The process of any preceding clause, wherein at least one R¹ comprises an aryl or heteroaryl group; optionally wherein at least one R¹ is -CH₂-pyrenyl.

Clause 24. The process of any preceding clause, wherein each R¹ is the same.

Clause 25. The process of any preceding clause, wherein each Y is -CH₂-.

Clause 26. The process of any preceding clause, wherein each R² is cyclohexyl.

Clause 27. The process of any preceding clause, wherein the process is performed anaerobically.

Clause 28. A system comprising:
(i) a catalyst of Formula (I) 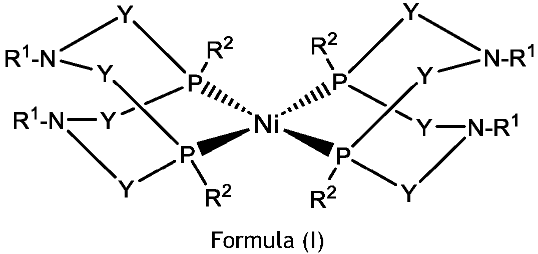 wherein:
   Y, each independently, is C(R³)_{2;} and R³, each independently, is H or C₁₋₃ alkyl;
   R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
   R², each independently, is a 5-6 membered cyclic group, wherein the cyclic group may be optionally substituted, and wherein the cyclic group may optionally include one or more heteroatoms N, O or S in its carbon skeleton;
(ii) a molecular reductant; and
(iii) a target compound comprising a nitrogen-containing functional group;
wherein the system is configured such that, in use, the nitrogen-containing functional group is reduced.

For the avoidance of doubt, insofar as is practicable any embodiment of a given aspect of the processes herein described may occur in combination with any other embodiment of the same aspect. In addition, insofar as is practicable it is to be understood that any preferred, typical or optional embodiment of any aspect of the processes herein described should also be considered as a preferred, typical or optional embodiment of any other aspect.

The following Examples illustrate the invention. They do not, however, limit the invention in any way. In this regard, it is important to understand that the particular assays used in the Examples section are designed only to provide an indication of the efficacy of the method of the invention.

### EXAMPLES

### Example 1 - Preparation of NiArg/C

In an anaerobic glovebox under a N₂ atmosphere, carbon black (Vulcan XC72R) is sonicated in sodium phosphate buffer (100 mM, pH 6) for 1 h to produce a slurry (20 mg/mL). For one 2 mL scale reaction (10 mM substrate), this suspension of carbon (180 µL, 0.0035 mg) is combined with NiArg (12 µL, 5 mM, 0.000089 mg, 6×10⁻⁵ mmol). The mixture is left on ice in an anaerobic glovebox for 1 h, after which it is centrifuged and the supernatant is discarded. The solid is resuspended in the same buffer (100 µL).

### Example 2 - Nitrobenzene reduction

Nitrobenzene, 10 mM in aqueous phosphate buffer (pH 6), was placed in a vial in a DrySyn OCTO reactor and Ni catalyst (NiArg/C or NiArg respectively, corresponding to 0.3 mol% complex to nitrobenzene and for NiArg/C 40:1 carbon to complex ratio by weight) was added. The reactor was connected to a flow of hydrogen (30 mL/min) in an anaerobic environment at room temperature and pressure for 24 hours or 48 hours.

Reaction samples were then analysed by 1H NMR (in the glovebox, the mixture is sampled and centrifuged, the supernatant is combined with D₂O). All NMR spectra were obtained using a Bruker Advance III HD nanobay (400 MHz) instrument, the samples contained the reaction mixture with 10% D₂O and a water suppression was applied. Data were processed using MestReNova.

Signals were referenced against appropriate peaks such as acetonitrile (δ = 2.06 ppm).

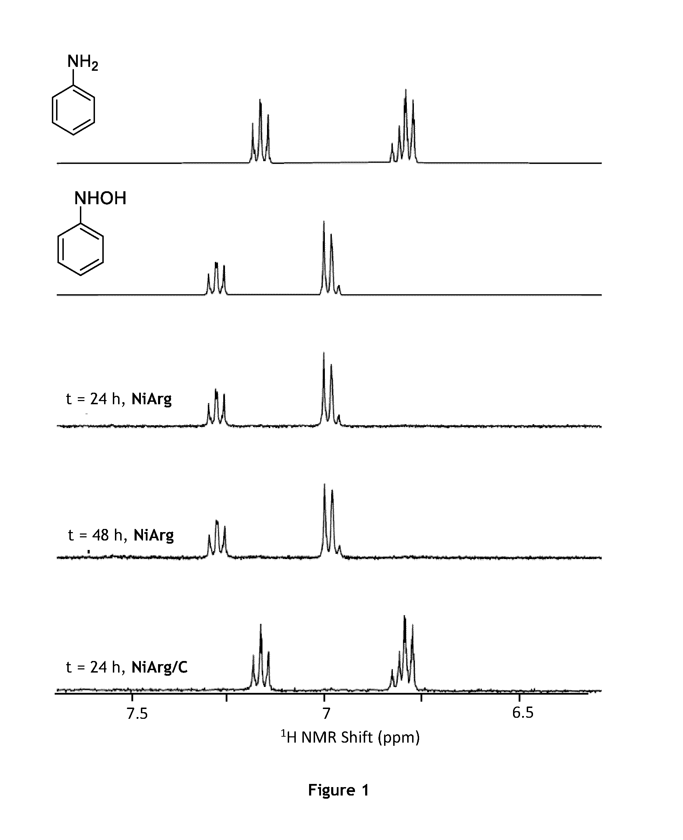

As shown in Figure 1, treatment of nitrobenzene with either NiArg or NiArg/C, respectively, yields N-phenylhydroxylamine or aniline, respectively, as identified by 1H nuclear magnetic resonance spectroscopy (NMR).

### Example 3 - Nitrobenzene reduction

The process of example 2 was repeated but with different carbon to catalyst ratios and catalyst loadings as set out in the table below:

| **Catalyst loading (mol/mol)** | **Carbon: Catalyst (w:w)** | **Product after 24 hours as measured by ¹H NMR relative integrals** | | |
|---|---|---|---|---|
| | | **Nitrobenzene** | **N-phenyl hydroxylamine** | **Aniline** |
| 0.1% | No carbon | 0 | 1 | 0 |
| 0.3% | No carbon | 0 | 1 | 0 |
| 0.3% | 5:1 | 0 | >0.95 | <0.05 |
| 0.3% | 10:1 | 0 | ~0.9 | -0.1 |
| 0.3% | 40:1 | 0 | 0 | 1 |

As shown by the results in the table, at low amounts of carbon only small amounts of aniline were observed after 24 h. To ensure full conversion to aniline, a 40:1 ratio was chosen.

### Example 4 - Nitrobenzene reduction mechanistic and kinetic study

Nitrobenzene, 10 mM in aqueous phosphate buffer (pH 6), was placed in a vial in a DrySyn OCTO reactor and Ni catalyst (NiArg/C or NiArg respectively) was added (corresponding to a catalyst loading of 0.3 mol% complex: nitrobenzene and where applicable a 40:1 carbon to complex ratio). The reactor was connected to a flow of hydrogen (30 mL/min) in an anaerobic environment at room temperature and pressure for 24 hours.

The reactions were sampled at intervals over the 24 hour reaction time.

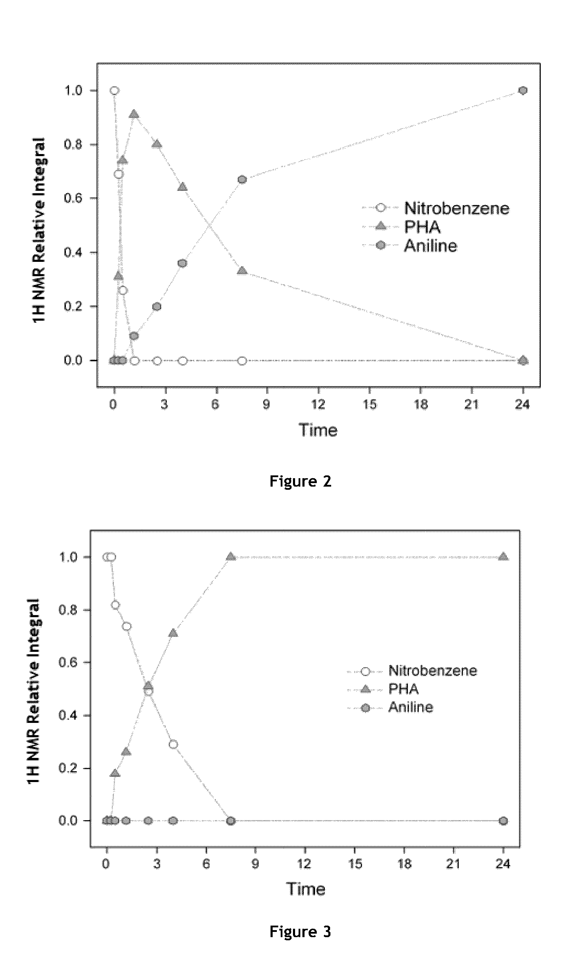

Figure 2 is a graph showing the relative integral (as measured by 1H NMR) of nitrobenzene, N-phenylhydroxylamine (PHA) and aniline as measured over 24 hours for a hydrogenation of nitrobenzene in the presence of NiArg/C.

Figure 3 is a graph showing the relative integral (as measured by 1H NMR) of nitrobenzene, N-phenylhydroxylamine (PHA) and aniline as measured over 24 hours for a hydrogenation of nitrobenzene in the presence of NiArg.

The PHA forms very quickly (within an hour when using NiArg/C), while subsequent conversion to the aniline occurs over the course of 24 h.

This is reflected in the relevant calculated turnover frequency (TOF) numbers. The addition of carbon black increased the TOF from 0.0075 s⁻¹ for NiArg to 0.013 s⁻¹ for NiArg/C, as calculated towards the PHA, which is in line with the assumption that the reaction occurs on the particle surface. The calculated TOF for NiArg/C, as calculated towards the aniline, was 0.0034 s⁻¹.

### Example 5 - Nitrobenzene reduction in water

Nitrobenzene, 10 mM in water (containing 5 v/v% acetonitrile), was placed in a vial in a DrySyn OCTO reactor and Ni catalyst (NiArg/C or NiArg respectively, corresponding to a catalyst loading of 0.5 mol% complex: nitrobenzene and where applicable a 40:1 carbon to complex ratio by weight) was added. The reactor was connected to a flow of hydrogen (30 mL/min) in an anaerobic environment at room temperature and pressure.

Reaction samples were then analysed by 1H NMR (in the glovebox, the mixture is sampled and centrifuged, the supernatant is combined with D₂O). All NMR spectra were obtained using a Bruker Advance III HD nanobay (400 MHz) instrument and a water suppression was applied. Data was processed using MestReNova.

Signals were referenced against appropriate peaks such as acetonitrile (δ = 2.06 ppm).

For the reaction towards the PHA using the NiArg catalyst, the system reached near full conversion within 24 or 48 hours. Conversion to the amine using the NiArg/C catalyst was slower than in the presence of buffer.

### Example 6 - Substituted nitrobenzene reduction

The respective substituted nitrobenzene (depicted below), 10 mM in aqueous phosphate buffer (pH 6), was placed in a vial in a DrySyn OCTO reactor and Ni catalyst (NiArg/C or NiArg respectively, corresponding to a catalyst loading of 0.3 mol% complex: nitrobenzene and where applicable a 40:1 carbon to complex ratio by weight) was added. The reactor was connected to a flow of hydrogen (30 mL/min) in an anaerobic environment at room temperature and pressure for 24 hours (unless stated otherwise below).

Reaction samples were then analysed by 1H NMR (in the glovebox, the mixture is sampled and centrifuged, the supernatant is combined with D₂O) and the relative integral of hydroxylamine : amine product determined.

| | | | | |
|---|---|---|---|---|
| | 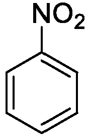 | 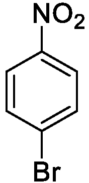 | 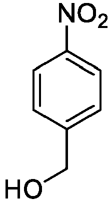 | 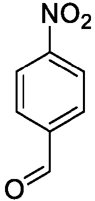 |
| **Catalyst** | **hydroxylamine** : **amine relative integral** | | | |
| NiArg | 1:0 | 1:0 | 1:0 | 1:0 |
| NiArg/C | 0:1 | 0:1 | 0:1 | 0:1^{≠} |
| | 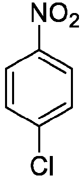 | 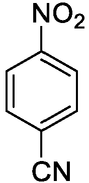 | 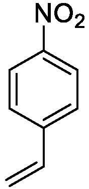 | 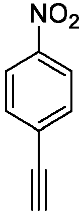 |
| **Catalyst** | **hydroxylamine** : **amine relative integral** | | | |
| NiArg | 1:0 | 1:0 | 1:0 | 1:0* |
| NiArg/C | 0:1 | 0:1 | 2:1 | 1.7:1* |
| | 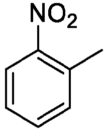 | 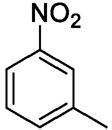 | 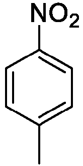 | 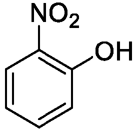 |
| **Catalyst** | **hydroxylamine** : **amine relative integral** | | | |
| NiArg | 1:0 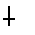 | 1:0 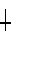 | 1:0 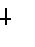 | 0:1 |
| NiArg/C | 0:1 | 0:1 | 0:1 | 0:1 |
| | 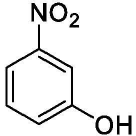 | 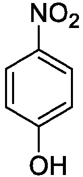 | 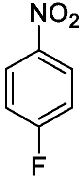 | |
| **Catalyst** | **hydroxylamine : amine relative integral** | | | |
| NiArg | 1:0 | 0:0.6^{‡} | 1:0 | |
| NiArg/C | 0:1 | 0:1 | 0:1 | |

| | | | | |
|---|---|---|---|---|
| *reaction in pH 8, 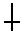 after 48 h, ‡ after 72 h | | | | |

### Example 7 - Hydrogenation of nitrohexane

Nitrohexane, 10 mM in aqueous phosphate buffer (pH 6), was placed in a vial in a DrySyn OCTO reactor and NiArg/C catalyst (0.5 mol% complex to nitrohexane, 40:1 carbon to catalyst ratio by weight) was added. The reactor was connected to a flow of hydrogen (30 mL/min) in an anaerobic environment at room temperature and pressure for 24 hours.

The reaction was then analysed by 1H NMR (in the glovebox, the mixture is sampled and centrifuged, the supernatant is combined with D₂O). All NMR spectra were obtained using a Bruker Advance III HD nanobay (400 MHz) instrument and a water suppression was applied.

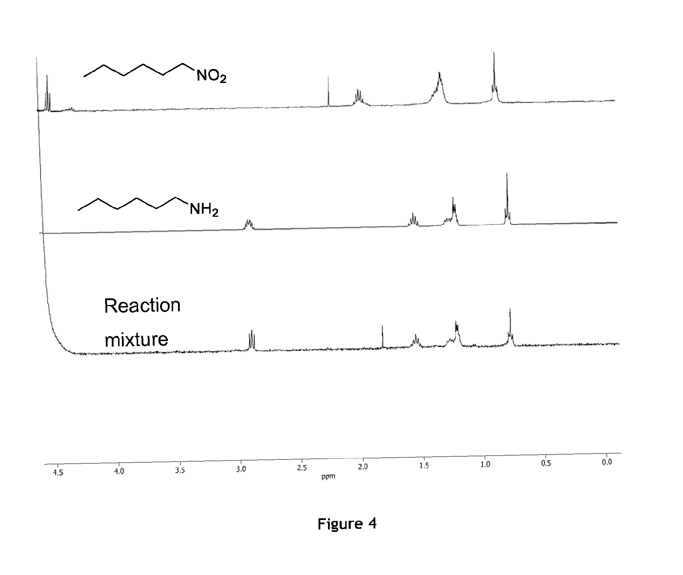

As shown in Figure 4, treatment of nitrohexane with NiArg/C yields the corresponding amine as identified by 1H nuclear magnetic resonance spectroscopy (NMR).

### Example 8 - Preparation of NiArg/NCNT

Nitrogen doped carbon nanotubes (NCNTs) were prepared following a published procedure described in Koós, A. A.; Dowling, M.; Jurkschat, K.; Crossley, A.; Grobert, N. Effect of the experimental parameters on the structure of nitrogen-doped carbon nanotubes produced by aerosol chemical vapour deposition. Carbon 2009, 47 (1), 30-37. DOI: https://doi.org/10.1016/j.carbon.2008.08.014. Briefly, benzylamine containing 5wt% ferrocene was fed into a piezo-driven aerosol generator which was then connected to a quartz tube within a tube furnace. The solution was aerosolized and fed into the preheated furnace (900 °C) under flow of argon gas. After 25 min, the furnace was isolated and allowed to cool to room temperature before the produced NCNTs were collected.

In the glovebox, nitrogen doped carbon nanotubes (NCNTs) are sonicated in sodium phosphate buffer (100 mM, pH 6) for 1 h to produce a slurry (20 mg/mL). For one 1 mL/10 mM scale reaction, this suspension (190 µL, 0.00367 mg) is combined with NiArg (5 µL, 10 mM, 5×10⁻⁵ mmol). The mixture is left on ice in the glovebox for 1 h, after which it is centrifuged and the supernatant is discarded. The solid is resuspended in the same buffer (100 µL).

### Example 9 - Hydrogenation of cyclo-nitrohexane

Cyclo-nitrohexane, 10 mM in aqueous phosphate buffer (pH 6), was placed into a vial with a pierced lid and NiArg/NCNT catalyst (0.5 mol% complex to cyclo-nitrohexane, 40:1 carbon to complex ratio by weight) was added. The vial placed inside a Buchi Tinyclave pressure vessel which was then filled with H₂ (the vessel was filled to 1 bar and vented 5x, then filled to 1 bar and set onto a stirplate for 4 hours.

The reaction was then analysed by 1H NMR (in the glovebox, the mixture is sampled and centrifuged, the supernatant is combined with D₂O). All NMR spectra were obtained using a Bruker Advance III HD nanobay (400 MHz) instrument and a water suppression was applied.

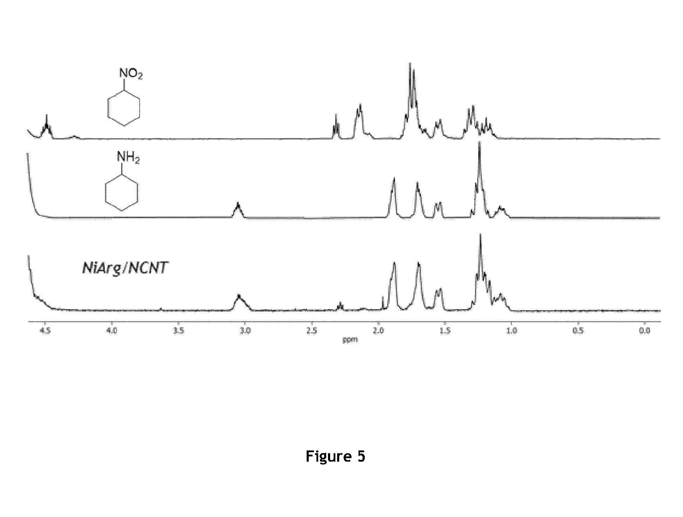

As shown in Figure 5, treatment of cyclo-nitrohexane with NiArg/NCNT catalyst yields the corresponding amine as identified by 1H nuclear magnetic resonance spectroscopy (NMR).

### Example 10 - One-pot catalyst prep from [Ni(H₂O)₆](BF₄)₂

Dutta, A.; Roberts, J. A. S.; Shaw, W. J. Arginine-Containing Ligands Enhance H2 Oxidation Catalyst Performance. Angewandte Chemie International Edition 2014, 53 (25), 6487-6491. DOI: https://doi.org/10.1002/anie.201402304 described details the synthesis of the ligands and the complex.

The P₂^{Cy}N₂^{Arg} ligand (4 eq) is immobilised on carbon black (VulcanX) as before by simply combining a carbon slurry with a solution of the ligand and leaving the mixture on ice for 1 h. The modified carbon, ligand/carbon (L/C), is collected and resuspended in acetonitrile. The [Ni(H₂O)₆](BF₄)₂ (1 eq) is added and the mixture is stirred overnight, before being collected, washed, and used as the catalyst in the hydrogenation of nitrobenzene.

Nitrobenzene, 10 mM in aqueous phosphate buffer (pH 6), was placed in a vial in a DrySyn OCTO reactor and the catalyst (0.5 mol% complex: nitrobenzene, 40:1 carbon to catalyst ratio by weight) was added. The reactor was connected to a flow of hydrogen (30 mL/min) in an anaerobic environment at room temperature and pressure for 24 hours.

This negates the need to isolate the NiArg/C complex and thus simplifies catalyst preparation.

For a control reaction, the equivalent amount of carbon was stirred with [Ni(H₂O)₆](BF₄)₂ in acetonitrile overnight, and then used as the catalyst in the hydrogenation of nitrobenzene. No product was observed.

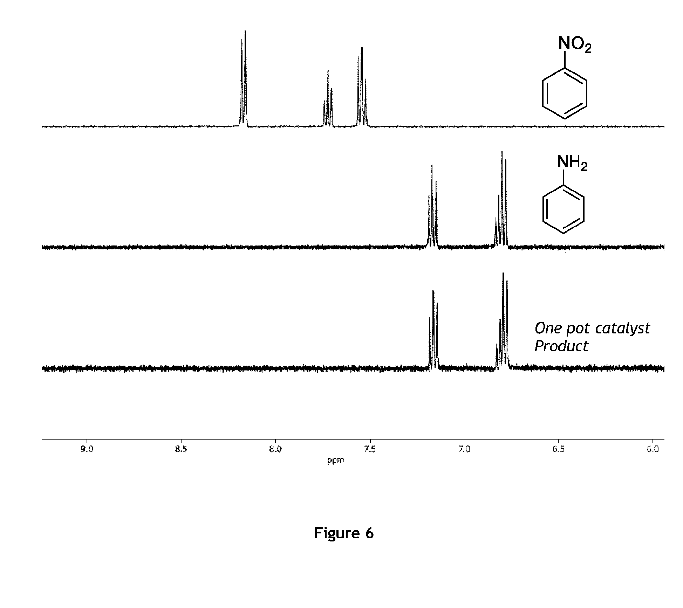

As shown in Figure 6, treatment of nitrobenzene with NiArg/C catalyst yields the corresponding amine as identified by 1H nuclear magnetic resonance spectroscopy (NMR).

### Example 11 - Electrochemistry of H₂ Oxidation at NiArg complex

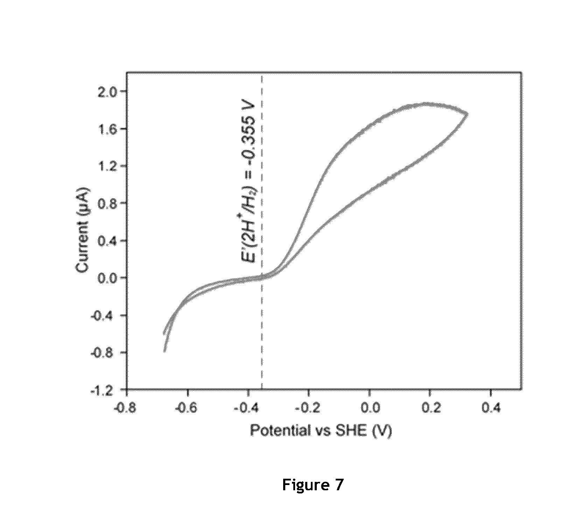

Cyclic voltammograms were obtained for the NiArg complex adsorbed onto a pyrolytic graphite edge-plane electrode under H₂ and electrode rotation at 3000 RPM, scan rate 1 mV/s. Dashed vertical line represents E'(2H⁺/H₂) at pH 6 and 1 bar H₂. Hydrogen oxidation catalysed by NiArg complex commences with very low overpotential as shown in Figure 7.

### Example 12 - Nitrobenzene reduction

Nitrobenzene, 10 mM in aqueous phosphate buffer (pH 6), was placed in a vial in a DrySyn OCTO reactor and Ni(-CH₂Py)/C was added. The reactor was connected to a flow of hydrogen (30 mL/min) in an anaerobic environment at room temperature and pressure for 24 hours.

The reaction was then analysed by 1H NMR (in the glovebox, the mixture is sampled and centrifuged, the supernatant is combined with D₂O).

Treatment of nitrobenzene with Ni(-CH₂Py)/C yields aniline the predominant product (0.09:1 PHA:Aniline ratio) as identified by 1H nuclear magnetic resonance spectroscopy (NMR).

## Claims

1. A process of reducing a nitrogen-containing functional group in a target compound, comprising contacting the target compound with a catalyst of Formula (I) in the presence of a molecular reductant: 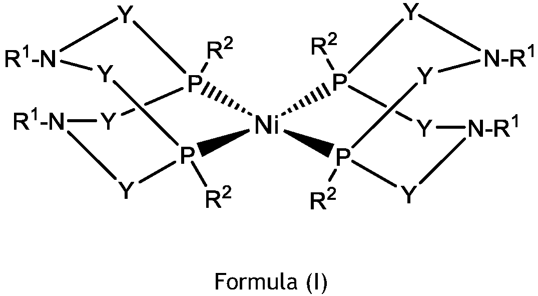 wherein:
Y, each independently, is -C(R³)₂-; and R³, each independently, is H or C₁₋₃ alkyl;
R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
R², each independently, is a 5-6 membered cyclic group, wherein the cyclic group may be optionally substituted, and wherein the cyclic group may optionally include one or more heteroatoms N, O or S in its carbon skeleton.

2. The process of claim 1, wherein the nitrogen-containing functional group is selected from the group consisting of nitro, hydroxylamine, azido, azo, azoxy and nitroso; optionally the nitrogen-containing functional group is a nitro group or a hydroxylamine functional group.

3. The process of claim 1 or claim 2, wherein the nitrogen-containing functional group is a nitro functional group.

4. The process of any preceding claim, wherein the process is performed in the presence of a protic solvent; optionally wherein the protic solvent comprises water or aqueous sodium phosphate.

5. The process of any preceding claim, wherein the catalyst is used in homogeneous form.

6. The process of any one of claims 1-4, wherein the catalyst is used in heterogeneous form; and the catalyst is supported on a support material and the weight ratio of the support material to the catalyst is 10 or less:1.

7. The process of any one of claims 1-6, wherein the nitrogen-containing functional group is a nitro functional group, comprising reducing the nitro functional group to form a hydroxylamine.

8. The process of any one of claims 1-4, wherein the catalyst is used in heterogeneous form.

9. The process of claim 8, wherein the catalyst is supported on a support material; and the weight ratio of the support material to the catalyst is 40 or more:1.

10. The process of claim 6 or claim 9, wherein:
(i) the support material is electronically conductive or semi-conductive;
(ii) the support material is electronically conductive or semi-conductive; and the support material comprises a carbon material; optionally wherein the carbon material comprises graphite, carbon nanotube(s), carbon black, activated carbon, carbon nanopowder, vitreous carbon, carbon fibre(s), carbon cloth, carbon felt, carbon paper, graphene, highly oriented pyrolytic graphite, pyrolytic graphite, carbon quantum dots, doped or surface-modified carbon or doped diamond;
(iii) the support material is electronically conductive or semi-conductive; and the support material comprises carbon black or nitrogen-doped carbon nanotubes; and/or
(iv) the catalyst is adsorbed to the support material.

11. The process of claim 9 or claim 10, wherein the nitrogen-containing functional group is a nitro functional group, comprising reducing the nitro functional group to form an amine.

12. The process of any preceding claim, wherein:
(i) the molecular reductant is H₂;
(ii) the molecular reductant is H₂; and the H₂ is provided by adding gaseous H₂ or a gas mixture comprising gaseous H₂ into a reaction vessel in which the process is performed; optionally wherein the gaseous H₂ or gas mixture are provided at atmospheric pressure; and/or
(iii) the process is performed anaerobically.

13. The process of any preceding claim, wherein:
(i) the target compound comprises two nitro functional groups;
(ii) the target compound comprises one nitro functional group;
(iii) the target compound is a nitroaromatic compound; or
(iv) the target compound is an aliphatic compound comprising a nitro group; optionally wherein the target compound is an alkyl compound comprising a nitro group or a cycloalkyl compound comprising a nitro group.

14. The process of any preceding claim, wherein:
(i) at least one R' is a group of Formula (II): 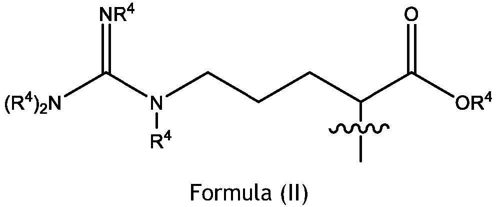 wherein R⁴, each independently, is H or C₁₋₃ alkyl; optionally wherein each R⁴ is H;
(ii) at least one R' comprises a guanidino group; optionally wherein at least one R¹ is argininyl;
(iii) at least one R¹ comprises an aryl or heteroaryl group; optionally wherein at least one R¹ is -CH₂-pyrenyl;
(iv) each R¹ is the same;
(v) each Y is -CH₂-; or
(vi) each R² is cyclohexyl.

15. A system comprising:
(i) a catalyst of Formula (I) 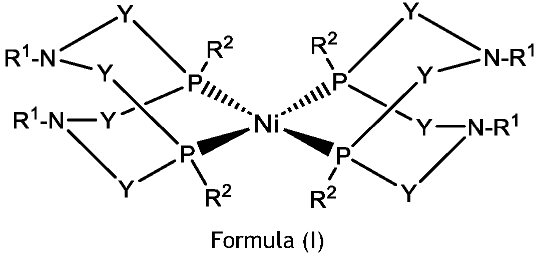 wherein:
Y, each independently, is C(R³)_{2;} and R³, each independently, is H or C₁₋₃ alkyl;
R¹, each independently, is a saturated or unsaturated C₁-C₃₀ hydrocarbyl group, wherein the hydrocarbyl group may be straight-chained or branched, or be or include cyclic groups, wherein the hydrocarbyl group may optionally be substituted, and wherein the hydrocarbyl group may optionally include one or more heteroatoms N, O or S in its carbon skeleton;
R², each independently, is a 5-6 membered cyclic group, wherein the cyclic group may be optionally substituted, and wherein the cyclic group may optionally include one or more heteroatoms N, O or S in its carbon skeleton;
(ii) a molecular reductant; and
(iii) a target compound comprising a nitrogen-containing functional group;
wherein the system is configured such that, in use, the nitrogen-containing functional group is reduced.
